# EUROPEAN PATENT APPLICATION

(11) **EP 4 850 701 A1**
(43) Date of publication of application: **09.12.2026**
(21) Application number: 25747931.1
(22) Date of filing: 26.01.2025
(51) Int. Cl.: A61K 47/68, A61K 39/395, A61K 31/537, A61K 31/555, A61K 31/7068, A61K 31/475, A61K 31/675, A61K 31/704, A61K 31/7048, A61K 31/573, A61P 35/00

(54) **ANTI-CD79B ANTIBODY-DRUG CONJUGATE COMBINED WITH IMMUNOTHERAPEUTIC AGENT AND CHEMOTHERAPEUTIC AGENT FOR TREATING TUMOR**

(30) Priority: 02.02.2024 CN 202410150219; 22.10.2024 CN 202411472550
(71) Applicant: Jiangsu Hengrui Pharmaceuticals Co., Ltd., Lianyungang, Jiangsu 222047 (CN)
(72) Inventor: XIAO, Zhenyu, Lianyungang, Jiangsu 222047 (CN)
(74) Representative: Potter Clarkson
(86) International application number: PCT/CN2025/075142
(87) International publication number: WO 2025/162364

(57) **Abstract**

The present invention relates to an anti-CD79b antibody-drug conjugate combined with an immunotherapeutic agent and a chemotherapeutic agent for treating a tumor, and in particular to a use of the anti-CD79b antibody-drug conjugate combined with the immunotherapeutic agent and/or the chemotherapeutic agent in the preparation of a drug for treating a tumor.

## Description

The present disclosure claims priority to Application No. CN2024101502193 and Application No. CN2024114725503, which are incorporated herein by reference.

### TECHNICAL FIELD

The present disclosure pertains to the field of pharmaceuticals and relates to a method for treating a tumor with an ANTI-CD79b ANTIBODY-DRUG CONJUGATE COMBINED WITH AN IMMUNOTHERAPEUTIC AGENT AND/OR A CHEMOTHERAPEUTIC AGENT and pharmaceutical use thereof.

### BACKGROUND

The B-cell receptor (BCR) complex is the most dominant molecule on the surface of B cells. The BCR complex consists of a membrane immunoglobulin (mIg) that recognizes and binds to an antigen and a heterodimer of Igα (CD79a) and Igβ (CD79b) that transmits antigen stimulation signals. Igα and Igβ are a 47 kDa glycoprotein and a 37 kDa glycoprotein, respectively, and are members of the immunoglobulin superfamily. The genes encoding Igα and Igβ are referred to as mb-1 and B29, respectively. Igα and Igβ each contain an Ig-like domain at the amino-terminus of their extracellular region. Both Igα and Igβ can be used as substrates for protein tyrosine kinases and participate in BCR signal transduction. The BCR is widely expressed on B-cell lymphoma and normal B cells. CD79b is highly specific to the B-cell lineage, expressed on various types of B-cell NHL at relatively high levels, and has a mechanism of action of killing B-cell tumors extensively. Moreover, preclinical studies have also verified that using CD79b as a binding site is more efficient than targeting CD79a. Therefore, the development of drugs targeting CD79b is expected to become an effective means for treating B-cell lymphoma, especially DLBCL.

Polatuzumab vedotin (Polivy), developed by Roche, is an ADC targeting CD79b. It has been approved by both the NMPA and FDA for use in combination with rituximab and bendamustine (BR) for R/R DLBCL, and in combination with rituximab, cyclophosphamide, doxorubicin, and prednisone (R-CHP) for previously untreated DLBCL. However, clinical results show that polatuzumab vedotin has relatively high neurotoxicity, which limits its clinical administration window. Therefore, the development of safe and effective new treatment regimens for the CD79b target remains an important problem to be solved in the art.

### SUMMARY

The present disclosure provides a method for treating a tumor with an anti-CD79b antibody-drug conjugate combined with an immunotherapeutic agent and/or a chemotherapeutic agent and pharmaceutical use thereof.

In a first aspect, the present disclosure provides use of an anti-CD79b antibody-drug conjugate in combination with an immunotherapeutic agent in the manufacture of a medicament for treating a tumor.

In a second aspect, the present disclosure provides use of an anti-CD79b antibody-drug conjugate in combination with a chemotherapeutic agent in the manufacture of a medicament for treating a tumor.

In a third aspect, the present disclosure provides use of an anti-CD79b antibody-drug conjugate in combination with an immunotherapeutic agent and a chemotherapeutic agent in the manufacture of a medicament for treating a tumor.

In a fourth aspect, the present disclosure provides use of a combination of an anti-CD79b antibody-drug conjugate and an immunotherapeutic agent in the manufacture of a medicament for treating a tumor.

In a fifth aspect, the present disclosure provides use of a combination of an anti-CD79b antibody-drug conjugate and a chemotherapeutic agent in the manufacture of a medicament for treating a tumor.

In a sixth aspect, the present disclosure provides use of a combination of an anti-CD79b antibody-drug conjugate, an immunotherapeutic agent, and a chemotherapeutic agent in the manufacture of a medicament for treating a tumor.

In a seventh aspect, the present disclosure provides use of a pharmaceutical composition in the manufacture of a medicament for treating a tumor, wherein the pharmaceutical composition comprises: 1) an anti-CD79b antibody-drug conjugate and an immunotherapeutic agent, 2) an anti-CD79b antibody-drug conjugate and a chemotherapeutic agent, or 3) an anti-CD79b antibody-drug conjugate, an immunotherapeutic agent, and a chemotherapeutic agent.

In an eighth aspect, the present disclosure provides use of an anti-CD79b antibody-drug conjugate for treating a subject having a tumor, wherein the subject is further given: (1) an immunotherapeutic agent, (2) a chemotherapeutic agent, or (3) an immunotherapeutic agent and a chemotherapeutic agent.

In a ninth aspect, the present disclosure provides use of an immunotherapeutic agent and/or a chemotherapeutic agent for treating a subject having a tumor, wherein the subject is further given an anti-CD79b antibody-drug conjugate.

In a tenth aspect, the present disclosure provides a pharmaceutical composition comprising any one of the following:
1) an anti-CD79b antibody-drug conjugate and an immunotherapeutic agent, 2) an anti-CD79b antibody-drug conjugate and a chemotherapeutic agent, and 3) an anti-CD79b antibody-drug conjugate, an immunotherapeutic agent, and a chemotherapeutic agent. In an eleventh aspect, the present disclosure provides a method for treating a tumor, comprising administering to a subject in need thereof a therapeutically effective amount of: 1) an anti-CD79b antibody-drug conjugate and an immunotherapeutic agent, 2) an anti-CD79b antibody-drug conjugate and a chemotherapeutic agent, 3) an anti-CD79b antibody-drug conjugate, an immunotherapeutic agent, and a chemotherapeutic agent, or 4) an anti-CD79b antibody-drug conjugate.

In a twelfth aspect, the present disclosure provides a method for palliating an adverse reaction caused by an anti-CD79b antibody-drug conjugate in a subject, comprising administering to a subject in need thereof: 1) an anti-CD79b antibody-drug conjugate and an immunotherapeutic agent, 2) an anti-CD79b antibody-drug conjugate and a chemotherapeutic agent, 3) an anti-CD79b antibody-drug conjugate, an immunotherapeutic agent, and a chemotherapeutic agent, or 4) an anti-CD79b antibody-drug conjugate.

In some embodiments, the adverse reaction is neurotoxicity. In some embodiments, the adverse reaction is peripheral neurotoxicity.

In some embodiments, the tumor is B-cell lymphoma.

In some embodiments, the tumor is follicular lymphoma.

In some embodiments, the tumor is marginal zone lymphoma.

In some embodiments, the tumor is diffuse large B-cell lymphoma. In some embodiments, the diffuse large B-cell lymphoma comprises *de novo* DLBCL and indolent lymphoma-transformed DLBCL.

In some embodiments, the subject having the tumor has received at least one prior line of anti-tumor therapy.

In some embodiments, the subject having the tumor has received at least one prior line of anti-tumor therapy, and did not achieve a response after the last line of therapy or experienced disease progression and did not achieve a response after treatment or experienced disease progression after treatment.

Illustratively, the subject has received at least one prior line of anti-tumor therapy, achieved a response during treatment, and relapsed 6 months or more after completing treatment; or the subject has received at least one prior line of anti-tumor therapy and achieved no significant response during treatment, or relapsed within 6 months of completing treatment.

In some embodiments, the subject having the tumor is a treatment-naive patient who has not received anti-tumor therapy.

In some embodiments, the subject has diffuse large B-cell lymphoma, and the diffuse large B-cell lymphoma is relapsed or refractory diffuse large B-cell lymphoma.

Illustratively, the subject with relapsed or refractory diffuse large B-cell lymphoma must have received ≥1 prior line of systemic anti-tumor therapy, and did not achieve a response after the last line of therapy or experienced disease progression after treatment:
relapsed: achieved a response during treatment, and relapsed 6 months or more after completing treatment;
refractory: achieved no significant response during treatment, or relapsed within 6 months of completing treatment.

In some embodiments, the subject has Lugano stage I-II or III-IV disease. In some embodiments, the subject has Lugano stage I-II or III-IV diffuse large B-cell lymphoma.

In some embodiments, the subject has an IPI score of 0-1, 2, 3, or 4-5. In some embodiments, the subject has diffuse large B-cell lymphoma with an IPI score of 0-1, 2, 3, or 4-5.

In some embodiments, the subject with diffuse large B-cell lymphoma has not received prior lymphoma-related systemic anti-tumor therapy.

In the present disclosure, systemic anti-tumor treatment regimens are well known to those skilled in the art, e.g., treatment regimens disclosed in treatment guidelines approved by the supervision department. For example, the treatment regimens include, but are not limited to, the latest detailed information provided in the NCCN guidelines and the CSCO guidelines regarding standard treatment regimens for various types of cancer.

### Immunotherapeutic Agent and Chemotherapeutic Agent

In some embodiments, the immunotherapeutic agent is an anti-CD20 antibody. In some specific embodiments, the anti-CD20 antibody is rituximab. In some other embodiments, the anti-CD20 antibody may also be obinutuzumab, ofatumumab, ublituximab, or the like. In some embodiments, the chemotherapeutic agent is selected from the group consisting of any one or any combination (any 2, 3, 4, 5, 6, 7, 8, or the like) of the following: gemcitabine, a platinum-based drug, cyclophosphamide, an anthracycline (e.g., doxorubicin), a vinca alkaloid (e.g., vincristine), prednisone, ifosfamide, and etoposide phosphate.

In some embodiments, the platinum-based drug is selected from the group consisting of oxaliplatin and carboplatin. In some embodiments, the platinum-based drug may also be a drug of another type, e.g., cisplatin, nedaplatin, or lobaplatin.

In some embodiments, the chemotherapeutic agent is selected from the group consisting of any one of the following combinations:
(a) gemcitabine and oxaliplatin;
(b) cyclophosphamide, an anthracycline (e.g., doxorubicin), a vinca alkaloid (e.g., vincristine), and prednisone;
(c) ifosfamide, carboplatin, and etoposide phosphate; and
(d) ifosfamide and carboplatin.

In some embodiments, the chemotherapeutic agent is selected from the group consisting of any one of the following combinations:
(a) gemcitabine and oxaliplatin;
(b) cyclophosphamide, doxorubicin, vincristine, and prednisone;
(c) ifosfamide, carboplatin, and etoposide phosphate; and
(d) ifosfamide and carboplatin.

In some embodiments, the anthracycline may also be substituted with another chemotherapeutic drug of the same class with comparable efficacy; for example, epirubicin is substituted for doxorubicin. In some embodiments, the vinca alkaloid may be substituted with another chemotherapeutic drug of the same class with comparable efficacy. For example, vindesine is substituted for vincristine.

In some embodiments, an administration cycle of any one of the chemotherapeutic agents is preferably every 1 week, every 2 weeks, every 3 weeks, every 4 weeks, every 6 weeks, or every 8 weeks.

### <R-Gemox Regimen>

In some embodiments, the combination of the immunotherapeutic agent and the chemotherapeutic agent is: rituximab, gemcitabine, and oxaliplatin.

### <R-CHOP Regimen>

In some embodiments, the combination of the immunotherapeutic agent and the chemotherapeutic agent is: rituximab, cyclophosphamide, an anthracycline (e.g., doxorubicin), a vinca alkaloid (e.g., vincristine), and prednisone.

In some embodiments, the combination of the immunotherapeutic agent and the chemotherapeutic agent is: rituximab, cyclophosphamide, doxorubicin, vincristine, and prednisone.

In some embodiments, the combination of the immunotherapeutic agent and the chemotherapeutic agent is: rituximab, cyclophosphamide, doxorubicin liposome, vincristine, and prednisone.

### <R-ICE Regimen>

In some embodiments, the combination of the immunotherapeutic agent and the chemotherapeutic agent is: rituximab, ifosfamide, carboplatin, and etoposide phosphate.

### <R-IC Regimen>

In some embodiments, the combination of the immunotherapeutic agent and the chemotherapeutic agent is: rituximab, ifosfamide, and carboplatin.

### Combination of Anti-CD79b Antibody-Drug Conjugate, Immunotherapeutic Agent, and Chemotherapeutic Agent

In some embodiments, a combination of an anti-CD79b antibody-drug conjugate, an immunotherapeutic agent, and a chemotherapeutic agent is selected from the group consisting of any one of the following combinations:
(a) the anti-CD79b antibody-drug conjugate, rituximab, gemcitabine, and oxaliplatin;
(b) the anti-CD79b antibody-drug conjugate, rituximab, cyclophosphamide, an anthracycline (e.g., doxorubicin), a vinca alkaloid (e.g., vincristine), and prednisone;
(c) the anti-CD79b antibody-drug conjugate, rituximab, cyclophosphamide, doxorubicin, vincristine, and prednisone;
(d) the anti-CD79b antibody-drug conjugate, rituximab, ifosfamide, carboplatin, and etoposide phosphate; and
(e) the anti-CD79b antibody-drug conjugate, rituximab, and ifosfamide.

In some embodiments, the doxorubicin is doxorubicin liposome. In some embodiments, the anthracycline may also be substituted with another chemotherapeutic drug of the same class with comparable efficacy; for example, epirubicin is substituted for doxorubicin.In some embodiments, the vinca alkaloid may be substituted with another chemotherapeutic drug of the same class with comparable efficacy. For example, vindesine is substituted for vincristine.

In some embodiments, the subject is a subject who has not received prior lymphoma-related systemic anti-tumor therapy, and the subject is given the following pharmaceutical combination:
an anti-CD79b antibody-drug conjugate, rituximab, cyclophosphamide, an anthracycline (e.g., doxorubicin), a vinca alkaloid (e.g., vincristine), and prednisone.

In some embodiments, the subject has received ≥1 prior line of systemic anti-tumor therapy, and the subject is given the following pharmaceutical combination:
an anti-CD79b antibody-drug conjugate, rituximab, gemcitabine, and oxaliplatin;
an anti-CD79b antibody-drug conjugate, rituximab, ifosfamide, carboplatin, and etoposide phosphate; or
an anti-CD79b antibody-drug conjugate, rituximab, and ifosfamide.

In some embodiments, the subject has not received prior treatment with gemcitabine, and the subject is given the following pharmaceutical combination: an anti-CD79b antibody-drug conjugate, rituximab, gemcitabine, and oxaliplatin.

In some embodiments, the subject is a subject with relapsed or refractory DLBCL who has not received prior treatment with gemcitabine, and the subject is given the following pharmaceutical combination: an anti-CD79b antibody-drug conjugate, rituximab, gemcitabine, and oxaliplatin.

In some embodiments, the subject has not received prior treatment with ifosfamide, and the subject is given the following pharmaceutical combination: an anti-CD79b antibody-drug conjugate, rituximab, ifosfamide, carboplatin, and etoposide phosphate; or an anti-CD79b antibody-drug conjugate, rituximab, and ifosfamide.

In some embodiments, the subject is a subject with relapsed or refractory DLBCL who has not received prior treatment with ifosfamide, and the subject is given the following pharmaceutical combination: an anti-CD79b antibody-drug conjugate, rituximab, ifosfamide, carboplatin, and etoposide phosphate; or an anti-CD79b antibody-drug conjugate, rituximab, and ifosfamide.

### Administration Regimen

In some embodiments, the use or method according to any one of the foregoing uses any one of the following administration regimens:
(1) about 1.0 mg/kg to about 10 mg/kg of the anti-CD79b antibody-drug conjugate, about 1 mg/m² to about 1000 mg/m² of rituximab, about 5 mg/m² to about 2000 mg/m² of gemcitabine, and about 1-500 mg/m² of oxaliplatin;
   and an administration cycle is every 1 week, every 2 weeks, every 3 weeks, every 4 weeks, every 6 weeks, or every 8 weeks, preferably every 3 weeks;
(2) about 1.0 mg/kg to about 10 mg/kg of the anti-CD79b antibody-drug conjugate, about 1 mg/m² to about 1000 mg/m² of rituximab, about 1 mg/m² to about 1000 mg/m² of cyclophosphamide, about 1 mg/m² to about 500 mg/m² of an anthracycline, about 0.1 mg/m² to about 100 mg/m² of a vinca alkaloid, and about 1 mg/time to about 1000 mg/time of prednisone;
   and an administration cycle is every 1 week, every 2 weeks, every 3 weeks, every 4 weeks, every 6 weeks, or every 8 weeks, preferably every 3 weeks;
(3) about 1.0 mg/kg to about 10 mg/kg of the anti-CD79b antibody-drug conjugate, about 1 mg/m² to about 1000 mg/m² of rituximab, about 0.1 mg/m² to about 100 mg/m² of ifosfamide, about 1 mg to about 2000 mg of carboplatin, and about 1 mg/m²/time to about 500 mg/m²/time of etoposide phosphate;
   and an administration cycle is every 1 week, every 2 weeks, every 3 weeks, every 4 weeks, every 6 weeks, or every 8 weeks, preferably every 3 weeks;
(4) about 1.0 mg/kg to about 10 mg/kg of the anti-CD79b antibody-drug conjugate, about 1 mg/m² to about 1000 mg/m² of rituximab, about 0.1 mg/m² to about 100 mg/m² of ifosfamide, and about 1 mg to about 2000 mg of carboplatin;
   and an administration cycle is every 1 week, every 2 weeks, every 3 weeks, every 4 weeks, every 6 weeks, or every 8 weeks, preferably every 3 weeks;
(5) about 0.1 mg/kg, about 0.3 mg/kg, about 0.6 mg/kg, about 1.2 mg/kg, about 1.8 mg/kg, about 2.7 mg/kg, about 3.6 mg/kg, about 4.8 mg/kg, about 6.4 mg/kg, about 8.0 mg/kg, or about 10 mg/kg of the anti-CD79b antibody-drug conjugate, about 375 mg/m² of rituximab, about 1000 mg/m² of gemcitabine, and about 100 mg/m² of oxaliplatin;
   and an administration cycle is every 1 week, every 2 weeks, every 3 weeks, every 4 weeks, every 6 weeks, or every 8 weeks, preferably every 3 weeks;
(6) about 0.1 mg/kg, about 0.3 mg/kg, about 0.6 mg/kg, about 1.2 mg/kg, about 1.8 mg/kg, about 2.7 mg/kg, about 3.6 mg/kg, about 4.8 mg/kg, about 6.4 mg/kg, about 8.0 mg/kg, or about 10 mg/kg of the anti-CD79b antibody-drug conjugate, about 375 mg/m² of rituximab, about 750 mg/m² of cyclophosphamide, about 30-40 mg/m² of an anthracycline (e.g., doxorubicin), about 1.4 mg/m² of a vinca alkaloid (e.g., vincristine), and about 100 mg of prednisone;
   and an administration cycle is every 1 week, every 2 weeks, every 3 weeks, every 4 weeks, every 6 weeks, or every 8 weeks, preferably every 3 weeks;
(7) about 0.1 mg/kg, about 0.3 mg/kg, about 0.6 mg/kg, about 1.2 mg/kg, about 1.8 mg/kg, about 2.7 mg/kg, about 3.6 mg/kg, about 4.8 mg/kg, about 6.4 mg/kg, about 8.0 mg/kg, or about 10 mg/kg of the anti-CD79b antibody-drug conjugate, about 375 mg/m² of rituximab, about 5 mg/m² of ifosfamide, ≤800 mg of carboplatin, and about 100 mg/m² of etoposide phosphate;
   and an administration cycle is every 1 week, every 2 weeks, every 3 weeks, every 4 weeks, every 6 weeks, or every 8 weeks, preferably every 3 weeks; and
(8) about 0.1 mg/kg, about 0.3 mg/kg, about 0.6 mg/kg, about 1.2 mg/kg, about 1.8 mg/kg, about 2.7 mg/kg, about 3.6 mg/kg, about 4.8 mg/kg, about 6.4 mg/kg, about 8.0 mg/kg, or about 10 mg/kg of the anti-CD79b antibody-drug conjugate, about 375 mg/m² of rituximab, about 5 mg/m² of ifosfamide, and ≤800 mg of carboplatin;
   and an administration cycle is every 1 week, every 2 weeks, every 3 weeks, every 4 weeks, every 6 weeks, or every 8 weeks, preferably every 3 weeks.
   In some embodiments, the administration regimen is any one of the following:
(9) about 1.8 mg/kg, about 2.7 mg/kg, or about 3.6 mg/kg of the anti-CD79b antibody-drug conjugate, about 375 mg/m² of rituximab, about 1000 mg/m² of gemcitabine, and about 100 mg/m² of oxaliplatin;
   and an administration cycle is every 1 week, every 2 weeks, every 3 weeks, every 4 weeks, every 6 weeks, or every 8 weeks, preferably every 3 weeks;
(10) about 1.8 mg/kg, about 2.7 mg/kg, or about 3.6 mg/kg of the anti-CD79b antibody-drug conjugate, about 375 mg/m² of rituximab, about 750 mg/m² of cyclophosphamide, about 30-40 mg/m² of an anthracycline (e.g., doxorubicin), about 1.4 mg/m² of a vinca alkaloid (e.g., vincristine), and about 100 mg of prednisone;
   and an administration cycle is every 1 week, every 2 weeks, every 3 weeks, every 4 weeks, every 6 weeks, or every 8 weeks, preferably every 3 weeks;
(11) about 1.8 mg/kg, about 2.7 mg/kg, or about 3.6 mg/kg of the anti-CD79b antibody-drug conjugate, about 375 mg/m² of rituximab, about 5 mg/m² of ifosfamide, ≤800 mg of carboplatin, and about 100 mg/m² of etoposide phosphate;
   and an administration cycle is every 1 week, every 2 weeks, every 3 weeks, every 4 weeks, every 6 weeks, or every 8 weeks, preferably every 3 weeks; and
(12) about 1.8 mg/kg, about 2.7 mg/kg, or about 3.6 mg/kg of the anti-CD79b antibody-drug conjugate, about 375 mg/m² of rituximab, about 5 mg/m² of ifosfamide, and ≤800 mg of carboplatin;
   and an administration cycle is every 1 week, every 2 weeks, every 3 weeks, every 4 weeks, every 6 weeks, or every 8 weeks, preferably every 3 weeks.

In some embodiments, rituximab is administered before the anti-CD79b antibody-drug conjugate.

In some embodiments, the administration regimen is any one of the following:
(13) with 3 weeks as one administration cycle, about 1 mg/m² to about 1000 mg/m² of rituximab is administered on day 1 of each cycle, and about 1.0 mg/kg to about 10 mg/kg of the anti-CD79b antibody-drug conjugate, about 5 mg/m² to about 2000 mg/m² of gemcitabine, and about 1-500 mg/m² of oxaliplatin are administered on day 2 of each cycle;
(14) with 3 weeks as one administration cycle, about 1 mg/m² to about 1000 mg/m² of rituximab is administered on day 1 of each cycle, about 1.0 mg/kg to about 10 mg/kg of the anti-CD79b antibody-drug conjugate, about 1 mg/m² to about 1000 mg/m² of cyclophosphamide, about 1 mg/m² to about 500 mg/m² of an anthracycline, and about 0.1 mg/m² to about 100 mg/m² of a vinca alkaloid are administered on day 2 of each cycle, and about 1 mg/time to about 1000 mg/time of prednisone is administered on days 2, 3, 4, 5, and 6 of each cycle;
(15) with 3 weeks as one administration cycle, about 1 mg/m² to about 1000 mg/m² of rituximab is administered on day 1 of each cycle, about 1.0 mg/kg to about 10 mg/kg of the anti-CD79b antibody-drug conjugate is administered on day 2 of each cycle, about 0.1 mg/m² to about 100 mg/m² of ifosfamide and about 1 mg to about 2000 mg of carboplatin are administered on day 3 of each cycle, and about 1 mg/m²/time to about 500 mg/m²/time of etoposide phosphate is administered on days 2, 3, and 4 of each cycle; and
(16) with 3 weeks as one administration cycle, about 1 mg/m² to about 1000 mg/m² of rituximab is administered on day 1 of each cycle, about 1.0 mg/kg to about 10 mg/kg of the anti-CD79b antibody-drug conjugate is administered on day 2 of each cycle, and about 0.1 mg/m² to about 100 mg/m² of ifosfamide and about 1 mg to about 2000 mg of carboplatin are administered on day 3 of each cycle.

In some embodiments, the administration regimen is any one of the following:
(17) with 3 weeks as one administration cycle, about 50 mg/m² to about 500 mg/m² of rituximab is administered on day 1 of each cycle, and about 1.0 mg/kg to about 10 mg/kg of the anti-CD79b antibody-drug conjugate, about 500 mg/m² to about 1500 mg/m² of gemcitabine, and about 50-150 mg/m² of oxaliplatin are administered on day 2 of each cycle;
(18) with 3 weeks as one administration cycle, about 50 mg/m² to about 500 mg/m² of rituximab is administered on day 1 of each cycle, about 1.0 mg/kg to about 10 mg/kg of the anti-CD79b antibody-drug conjugate, about 500 mg/m² to about 1000 mg/m² of cyclophosphamide, about 10 mg/m² to about 100 mg/m² of an anthracycline, and about 0.1 mg/m² to about 100 mg/m² of a vinca alkaloid are administered on day 2 of each cycle, and about 50 mg/time to about 200 mg/time of prednisone is administered on days 2, 3, 4, 5, and 6 of each cycle;
(19) with 3 weeks as one administration cycle, about 50 mg/m² to about 500 mg/m² of rituximab is administered on day 1 of each cycle, about 1.0 mg/kg to about 10 mg/kg of the anti-CD79b antibody-drug conjugate is administered on day 2 of each cycle, about 1 mg/m² to about 10 mg/m² of ifosfamide and about 10 mg to about 800 mg of carboplatin are administered on day 3 of each cycle, and about 50 mg/m²/time to about 150 mg/m²/time of etoposide phosphate is administered on days 2, 3, and 4 of each cycle; and
(20) with 3 weeks as one administration cycle, about 50 mg/m² to about 500 mg/m² of rituximab is administered on day 1 of each cycle, about 1.0 mg/kg to about 10 mg/kg of the anti-CD79b antibody-drug conjugate is administered on day 2 of each cycle, and about 1 mg/m² to about 10 mg/m² of ifosfamide and about 10 mg to about 800 mg of carboplatin are administered on day 3 of each cycle.

In some embodiments, the administration regimen is any one of the following:
(21) with 3 weeks as one administration cycle, about 375 mg/m² of rituximab is administered on day 1 of each cycle, and about 1.0 mg/kg to about 10 mg/kg of the anti-CD79b antibody-drug conjugate, about 1000 mg/m² of gemcitabine, and about 100 mg/m² of oxaliplatin are administered on day 2 of each cycle;
(22) with 3 weeks as one administration cycle, about 375 mg/m² of rituximab is administered on day 1 of each cycle, about 1.0 mg/kg to about 10 mg/kg of the anti-CD79b antibody-drug conjugate, about 750 mg/m² of cyclophosphamide, about 30-40 mg/m² of an anthracycline, and about 1.4 mg/m² of a vinca alkaloid (e.g., about 1.4 mg/m² of vincristine, whose maximum dose is 2 mg) are administered on day 2 of each cycle, and about 100 mg/time of prednisone is administered on days 2, 3, 4, 5, and 6 of each cycle;
(23) with 3 weeks as one administration cycle, about 375 mg/m² of rituximab is administered on day 1 of each cycle, about 1.0 mg/kg to about 10 mg/kg of the anti-CD79b antibody-drug conjugate is administered on day 2 of each cycle, about 5 mg/m² of ifosfamide and ≤800 mg of carboplatin are administered on day 3 of each cycle, and about 100 mg/m²/time of etoposide phosphate is administered on days 2, 3, and 4 of each cycle; and
(24) with 3 weeks as one administration cycle, about 375 mg/m² of rituximab is administered on day 1 of each cycle, about 1.0 mg/kg to about 10 mg/kg of the anti-CD79b antibody-drug conjugate is administered on day 2 of each cycle, and about 5 mg/m² of ifosfamide and ≤800 mg of carboplatin are administered on day 3 of each cycle.

In some embodiments, the administration regimen is any one of the following:
(25) with 3 weeks as one administration cycle, about 375 mg/m² of rituximab is administered on day 1 of each cycle, and about 1.8 mg/kg, about 2.7 mg/kg, or about 3.6 mg/kg of the anti-CD79b antibody-drug conjugate, about 1000 mg/m² of gemcitabine, and about 100 mg/m² of oxaliplatin are administered on day 2 of each cycle;
(26) with 3 weeks as one administration cycle, about 375 mg/m² of rituximab is administered on day 1 of each cycle, about 1.8 mg/kg, about 2.7 mg/kg, or about 3.6 mg/kg of the anti-CD79b antibody-drug conjugate, about 750 mg/m² of cyclophosphamide, about 30-40 mg/m² of an anthracycline, and about 1.4 mg/m² of a vinca alkaloid (e.g., about 1.4 mg/m² of vincristine, whose maximum dose is 2 mg) are administered on day 2 of each cycle, and about 100 mg/time of prednisone is administered on days 2, 3, 4, 5, and 6 of each cycle;
(27) with 3 weeks as one administration cycle, about 375 mg/m² of rituximab is administered on day 1 of each cycle, about 1.8 mg/kg, about 2.7 mg/kg, or about 3.6 mg/kg of the anti-CD79b antibody-drug conjugate is administered on day 2 of each cycle, about 5 mg/m² of ifosfamide and ≤800 mg of carboplatin are administered on day 3 of each cycle, and about 100 mg/m²/time of etoposide phosphate is administered on days 2, 3, and 4 of each cycle; and
(28) with 3 weeks as one administration cycle, about 375 mg/m² of rituximab is administered on day 1 of each cycle, about 1.8 mg/kg, about 2.7 mg/kg, or about 3.6 mg/kg of the anti-CD79b antibody-drug conjugate is administered on day 2 of each cycle, and about 5 mg/m² of ifosfamide and ≤800 mg of carboplatin are administered on day 3 of each cycle.

In some embodiments, in any one of the aforementioned administration regimens, the anti-CD79b antibody-drug conjugate, rituximab, gemcitabine, oxaliplatin, cyclophosphamide, the anthracycline, the vinca alkaloid, prednisone, ifosfamide, carboplatin, and etoposide phosphate are each independently shown below:

### <Anti-CD79b Antibody-Drug Conjugate>

In some embodiments, the anti-CD79b antibody-drug conjugate is administered at a dose of about 0.1 mg/kg to about 20 mg/kg, e.g., about 1.0 mg/kg to about 20 mg/kg, about 1.0 mg/kg to about 12 mg/kg, about 1.0 mg/kg to about 10.5 mg/kg, about 1.0 mg/kg to about 10 mg/kg, about 1.0 mg/kg to about 9 mg/kg, about 1.5 mg/kg to about 10.5 mg, about 1.5 mg/kg to about 9.0 mg/kg, about 3.0 mg/kg to about 9.0 mg/kg, about 4.5 mg/kg to about 9.0 mg/kg, about 6.0 mg/kg to about 9.0 mg/kg, about 7.5 mg/kg to about 9.0 mg/kg, about 1.5 mg/kg to about 7.5 mg/kg, about 1.5 mg/kg to about 6.5 mg/kg, about 1.0 mg/kg to about 4.8 mg/kg, about 3.0 mg/kg to about 7.5 mg/kg, or any range between these point values. In some embodiments, the anti-CD79b antibody-drug conjugate is administered at a dose selected from the group consisting of about 1.0 mg/kg, about 1.1 mg/kg, about 1.2 mg/kg, about 1.3 mg/kg, about 1.4 mg/kg, about 1.5 mg/kg, about 1.6 mg/kg, about 1.7 mg/kg, about 1.8 mg/kg, about 1.9 mg/kg, about 2.0 mg/kg, about 2.1 mg/kg, about 2.2 mg/kg, about 2.3 mg/kg, about 2.4 mg/kg, about 2.5 mg/kg, about 2.6 mg/kg, about 2.7 mg/kg, about 2.8 mg/kg, about 2.9 mg/kg, about 3.0 mg/kg, about 3.1 mg/kg, about 3.2 mg/kg, about 3.4 mg/kg, about 3.5 mg/kg, about 3.6 mg/kg, about 3.8 mg/kg, about 4.0 mg/kg, about 4.2 mg/kg, about 4.3 mg/kg, about 4.5 mg/kg, about 4.6 mg/kg, about 4.8 mg/kg, about 5.0 mg/kg, about 5.1 mg/kg, about 5.3 mg/kg, about 5.5 mg/kg, about 5.6 mg/kg, about 5.8 mg/kg, about 6.0 mg/kg, about 6.1 mg/kg, about 6.3 mg/kg, about 6.4 mg/kg, about 6.5 mg/kg, about 6.6 mg/kg, about 6.8 mg/kg, about 6.9 mg/kg, about 7.0 mg/kg, about 7.2 mg/kg, about 7.4 mg/kg, about 7.5 mg/kg, about 7.6 mg/kg, about 7.8 mg/kg, about 8.0 mg/kg, about 8.1 mg/kg, about 8.3 mg/kg, about 8.5 mg/kg, about 8.8 mg/kg, about 9.0 mg/kg, about 9.5 mg/kg, about 10.0 mg/kg, about 10.5 mg/kg, about 12 mg/kg, about 12.5 mg/kg, about 13 mg/kg, about 13.5 mg/kg, about 14 mg/kg, about 16 mg/kg, about 18 mg/kg, about 19 mg/kg, about 20 mg/kg, and any range between these point values.

In some embodiments, the anti-CD79b antibody-drug conjugate is administered at a frequency of once weekly, once every 2 weeks, once every 3 weeks, once every 4 weeks, once every 6 weeks, or once every 8 weeks. In some specific embodiments, the anti-CD79b antibody-drug conjugate is administered at a frequency of once every 3 weeks. In some embodiments, the administration regimen for the anti-CD79b antibody-drug conjugate is selected from the group consisting of any one of the following:
(a₁) being administered at a dose of about 0.1 mg/kg to about 20 mg/kg and at a frequency of once weekly, once every 2 weeks, once every 3 weeks, once every 4 weeks, once every 6 weeks, or once every 8 weeks;
(a₂) being administered at a dose of about 1.0 mg/kg to about 10.5 mg/kg and at a frequency selected from the group consisting of once weekly, once every 2 weeks, once every 3 weeks, once every 4 weeks, once every 6 weeks, and once every 8 weeks;
(a₃) being administered at a dose of about 1.0 mg/kg to about 9.0 mg/kg and at a frequency selected from the group consisting of once weekly, once every 2 weeks, once every 3 weeks, once every 4 weeks, once every 6 weeks, and once every 8 weeks;
(a₄) being administered at a dose of about 1.0 mg/kg to about 8.0 mg/kg and at a frequency selected from the group consisting of once weekly, once every 2 weeks, once every 3 weeks, once every 4 weeks, once every 6 weeks, and once every 8 weeks;
(a₅) being administered at a dose of about 1.0 mg/kg to about 6.0 mg/kg and at a frequency selected from the group consisting of once weekly, once every 2 weeks, once every 3 weeks, once every 4 weeks, once every 6 weeks, and once every 8 weeks;
(a₆) being administered at a dose of about 0.1 mg/kg, about 0.3 mg/kg, about 0.6 mg/kg, about 1.2 mg/kg, about 1.8 mg/kg, about 2.7 mg/kg, about 3.6 mg/kg, about 4.8 mg/kg, about 6.4 mg/kg, about 8.0 mg/kg, or about 10 mg/kg and at a frequency selected from the group consisting of once weekly, once every 2 weeks, once every 3 weeks, once every 4 weeks, once every 6 weeks, and once every 8 weeks;
(a₇) being administered at a dose of about 0.1 mg/kg, about 0.3 mg/kg, about 0.6 mg/kg, about 1.2 mg/kg, about 1.8 mg/kg, about 2.7 mg/kg, about 3.6 mg/kg, about 4.8 mg/kg, about 6.4 mg/kg, about 8.0 mg/kg, or about 10 mg/kg and at a frequency selected from the group consisting of once every 2 weeks, once every 3 weeks, and once every 4 weeks;
(a₈) the anti-CD79b antibody-drug conjugate is administered at a dose of about 2.7 mg/kg and at a frequency of once every 3 weeks;
(a₉) the anti-CD79b antibody-drug conjugate is administered at a dose of about 3.6 mg/kg and at a frequency of once every 3 weeks;
(a₁₀) the anti-CD79b antibody-drug conjugate is administered at a dose of about 4.8 mg/kg and at a frequency of once every 3 weeks;
(a₁₁) the anti-CD79b antibody-drug conjugate is administered at a dose of about 6.4 mg/kg and at a frequency of once every 3 weeks;
(a₁₂) the anti-CD79b antibody-drug conjugate is administered at a dose of about 8.0 mg/kg and at a frequency of once every 3 weeks;
(a₁₃) the anti-CD79b antibody-drug conjugate is administered at a dose of about 1.2 mg/kg and at a frequency of once every 3 weeks; and
(a₁₄) the anti-CD79b antibody-drug conjugate is administered at a dose of about 10.0 mg/kg and at a frequency of once every 3 weeks.

In some specific embodiments, in the administration regimen according to any one of the (a₁)-(a₇) described above, the anti-CD79b antibody-drug conjugate is administered at a frequency of once every 3 weeks.

In some embodiments, the anti-CD79b antibody-drug conjugate is derived from an antibody-drug conjugate of any structure in WO2023143347A1 and WO2022022508A1, and the structures and the preparation methods of the immunoconjugates and other related contents in the above patents are incorporated herein by reference.

In some embodiments, the anti-CD79b antibody-drug conjugate has a structure represented by the following formula: 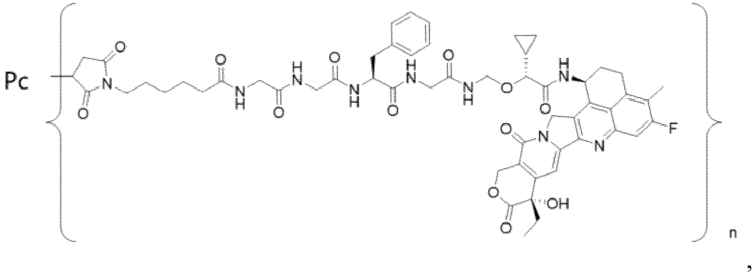 wherein n is 1 to 10, and n is a decimal or integer; Pc is an anti-CD79b antibody.

In some embodiments, n is a decimal or integer from 1 to 6. In some embodiments, n is a decimal or integer from 1 to 4. In some embodiments, n is 4.0±0.6. In some embodiments, n is 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, or 4.9. In the present disclosure, the term "antibody" is used in the broadest sense and encompasses a variety of antibody structures, including but not limited to monoclonal antibodies, polyclonal antibodies, multispecific antibodies (e.g., bispecific antibodies), full-length antibodies, or antigen-binding fragments thereof (also known as "antigen-binding moieties"), so long as they exhibit the desired antigen-binding activity.

In some embodiments, the anti-CD79b antibody is derived from an anti-CD79b antibody or an antigen-binding fragment thereof of any species in WO2022022508A1, and the antibody sequences, the preparation methods, and other related contents in the above patent are incorporated herein by reference.

In some embodiments, the anti-CD79b antibody comprises a heavy chain variable region (VH) and a light chain variable region (VL), wherein:
(1) the heavy chain variable region comprises a HCDR1, a HCDR2, and a HCDR3 set forth in SEQ ID NO: 11, SEQ ID NO: 6, and SEQ ID NO: 7, respectively, and/or the light chain variable region comprises a LCDR1, a LCDR2, and a LCDR3 set forth in SEQ ID NO: 8, SEQ ID NO: 9, and SEQ ID NO: 10, respectively;
(2) the heavy chain variable region comprises a HCDR1, a HCDR2, and a HCDR3 set forth in SEQ ID NO: 5, SEQ ID NO: 6, and SEQ ID NO: 7, respectively, and/or the light chain variable region comprises a LCDR1, a LCDR2, and a LCDR3 set forth in SEQ ID NO: 8, SEQ ID NO: 9, and SEQ ID NO: 10, respectively;
(3) the heavy chain variable region comprises a HCDR1, a HCDR2, and a HCDR3 from the sequence set forth in SEQ ID NO: 3, and/or the light chain variable region comprises a LCDR1, a LCDR2, and a LCDR3 from the sequence set forth in SEQ ID NO: 4.

In some specific embodiments, the anti-CD79b antibody comprises a heavy chain variable region (VH) and a light chain variable region (VL), wherein the heavy chain variable region comprises a HCDR1, a HCDR2, and a HCDR3 set forth in SEQ ID NO: 11, SEQ ID NO: 6, and SEQ ID NO: 7, respectively, and the light chain variable region comprises a LCDR1, a LCDR2, and a LCDR3 set forth in SEQ ID NO: 8, SEQ ID NO: 9, and SEQ ID NO: 10, respectively.

The CDRs are defined according to the Kabat, IMGT, Chothia, AbM, or Contact numbering scheme. In some specific embodiments, the CDRs are defined according to the Chothia numbering scheme.

The aforementioned CDR sequences are shown in the table below:

**Table 1. CDR sequences obtained according to the Chothia numbering scheme**

| Antibody CDR | mAb015 | hAb015-10 |
|---|---|---|
| HCDR1 | GSSFTSY (SEQ ID NO: 5) | GSSFSSY (SEQ ID NO: 11) |
| HCDR2 | FPRSGN (SEQ ID NO: 6) | SEQ ID NO: 6 |
| HCDR3 | GDLGDFDY (SEQ ID NO: 7) | SEQ ID NO: 7 |
| LCDR1 | RSSQSIVHSDGNTYFE (SEQ ID NO: 8) | SEQ ID NO: 8 |
| LCDR2 | KVSNRFS (SEQ ID NO: 9) | SEQ ID NO: 9 |
| LCDR3 | FQGSHVPWT (SEQ ID NO: 10) | SEQ ID NO: 10 |

In some embodiments, the anti-CD79b antibody comprises any 1, any 2, any 3, any 4, any 5, or 6 CDRs selected from the group consisting of a HCDR1 set forth in SEQ ID NO: 11 or 5 and a HCDR2, a HCDR3, a LCDR1, a LCDR2, and a LCDR3 set forth in SEQ ID NOs: 6-10.

In some embodiments, the anti-CD79b antibody is a murine-derived antibody, a chimeric antibody, a humanized antibody, a human antibody, or an antigen-binding fragment of any one of the foregoing. In some specific embodiments, the anti-CD79b antibody is selected from the group consisting of a humanized antibody and an antigen-binding fragment thereof.

In some embodiments, the VH of the anti-CD79b antibody comprises the amino acid sequence set forth in SEQ ID NO: 3 or an amino acid sequence having at least 80% or 90% sequence identity thereto, and the VL thereof comprises the amino acid sequence set forth in SEQ ID NO: 4 or an amino acid sequence having at least 80% or 90% sequence identity thereto.

In some embodiments, the VH of the anti-CD79b antibody comprises the amino acid sequence set forth in SEQ ID NO: 1 or an amino acid sequence having at least 80% or 90% sequence identity thereto, and the VL thereof comprises the amino acid sequence set forth in SEQ ID NO: 2 or an amino acid sequence having at least 80% or 90% sequence identity thereto.
> mAb015 VH: 
QVQLQQSGAELARPGASVKLSCKASGSSFTSYGINWVKQRTGQGLEWIGEIFPR
SGNTYYNEKFEGKATLTADKSSSTAYMELRSLTSEDSAVYFCAKGDLGDFDYW
GQGTTLTVSS
SEQ ID NO: 1
> mAb015 VL: 
DFLMTQTPLSLPVRLGDQASISCRSSQSIVHSDGNTYFEWYLQKPGQSPKLLIYK
VSNRFSGVPDRFSGSGSGTDFTLKISRVEAEDLGVYYCFQGSHVPWTFGGGTKL
EIK
SEQ ID NO: 2
> hAb015-10 VH: 
EVQLVQSGAEVKKPGSSVKVSCKASGSSFSSYGINWVKQAPGQGLEWIGEIFPR
SGNTYYNEKFEGRATLTADKSTSTAYMELRSLRSEDTAVYYCAKGDLGDFDYW
GQGTTVTVSS
SEQ ID NO: 3
> hAb015-10 VL:

DFVMTQTPLSLPVTPGEPASISCRSSQSIVHSDGNTYFEWYLQKPGQSPKLLIYK
VSNRFSGVPDRFSGSGSGTDFTLKISRVEAEDVGVYYCFQGSHVPWTFGGGTKV
EIK
SEQ ID NO: 4

In some embodiments, the anti-CD79b antibody comprises any 1 or 2 of the VH and VL described above.

In some embodiments, the anti-CD79b antibody further comprises antibody constant regions; for example, the heavy chain constant region among the antibody constant regions is selected from the group consisting of human IgG1, IgG2, IgG3, and IgG4 constant regions and variants thereof, and the light chain constant region among the antibody constant regions is selected from the group consisting of human antibody κ and λ chain constant regions and variants thereof.

The sequences of the light chain variable region and light chain constant region described above are combined to form a light chain sequence, and each heavy chain variable region is combined with the heavy chain constant region to form a heavy chain sequence. Exemplary humanized antibody sequences are shown below:
> hAb015-10 heavy chain: 
EVQLVQSGAEVKKPGSSVKVSCKASGSSFSSYGINWVKQAPGQGLEWIGEIFPR
SGNTYYNEKFEGRATLTADKSTSTAYMELRSLRSEDTAVYYCAKGDLGDFDYW
GQGTTVTVSSASTKGPSVFPLAPSSKSTSGGTAALGCLVKDYFPEPVTVSWNSG
ALTSGVHTFPAVLQSSGLYSLSSVVTVPSSSLGTQTYICNVNHKPSNTKVDKKVE
PKSCDKTHTCPPCPAPELLGGPSVFLFPPKPKDTLMISRTPEVTCVVVDVSHEDP
EVKFNWYVDGVEVHNAKTKPREEQYNSTYRVVSVLTVLHQDWLNGKEYKCK
VSNKALPAPIEKTISKAKGQPREPQVYTLPPSREEMTKNQVSLTCLVKGFYPSDI
AVEWESNGQPENNYKTTPPVLDSDGSFFLYSKLTVDKSRWQQGNVFSCSVMHE
ALHNHYTQKSLSLSPGK
SEQ ID NO: 12
> hAb015-10 light chain:

DFVMTQTPLSLPVTPGEPASISCRSSQSIVHSDGNTYFEWYLQKPGQSPKLLIYK
VSNRFSGVPDRFSGSGSGTDFTLKISRVEAEDVGVYYCFQGSHVPWTFGGGTKV
EIKRTVAAPSVFIFPPSDEQLKSGTASVVCLLNNFYPREAKVQWKVDNALQSGN

SQESVTEQDSKDSTYSLSSTLTLSKADYEKHKVYACEVTHQGLSSPVTKSFNRG
EC
SEQ ID NO: 13

In some embodiments, the anti-CD79b antibody comprises a heavy chain and a light chain, wherein the heavy chain variable region comprises the amino acid sequence set forth in SEQ ID NO: 12 or an amino acid sequence having at least 80% or 90% identity thereto, and the light chain variable region comprises the amino acid sequence set forth in SEQ ID NO: 13 or an amino acid sequence having at least 80% or 90% identity thereto.

In some embodiments, the anti-CD79b antibody comprises any 1 or 2 of the aforementioned heavy and light chains.

In the context of the present disclosure, "at least 80%" or "at least 90%" encompasses 80% or more, e.g., at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, and a numerical range between any two of these numbers.

In some embodiments, the route of administration of the anti-CD79b antibody-drug conjugate is oral administration, parenteral administration (including but not limited to intravenous injection, subcutaneous injection, and intramuscular injection), and percutaneous administration. In some specific embodiments, the route of administration of the anti-CD79b antibody-drug conjugate is intravenous injection.

In some embodiments, the anti-CD79b antibody-drug conjugate is formulated in an injectable form. Illustratively, the injectable form of the anti-CD79b antibody-drug conjugate is an injectable solution or freeze-dried powder comprising the anti-HCD79bER3 antibody-drug conjugate and one or more pharmaceutically acceptable auxiliary materials.

In some embodiments, the pharmaceutical composition comprising the anti-CD79b antibody-drug conjugate further comprises one or more of a buffer, a stabilizer, an osmotic pressure regulator, a surfactant, and a solvent.

In some embodiments, the buffer is selected from the group consisting of any one of an acetate buffer, a histidine salt buffer, a Tris-hydrochloride buffer, a Tris-citrate buffer, a phosphate buffer, or a succinate buffer. In some embodiments, the buffer is a histidine salt buffer or a succinate buffer. In some embodiments, the buffer is a histidine-hydrochloride buffer or a succinic acid-sodium succinate buffer.

In some embodiments, the surfactant is a nonionic surfactant. Illustratively, the surfactant is selected from the group consisting of poloxamer (e.g., poloxamer 188), polysorbate (e.g., polysorbate 20 (i.e., PS20) or polysorbate 80 (i.e., PS80)), poloxamer, triton, sodium dodecyl sulfonate, sodium lauryl sulfonate, sodium octyl glycoside, lauryl-sulfobetaine, myristyl-sulfobetaine, linoleyl-sulfobetaine, stearyl-sulfobetaine, lauryl-sarcosine, myristyl-sarcosine, linoleyl-sarcosine, stearyl-sarcosine, linoleyl-betaine, myristyl-betaine, cetyl-betaine, lauramidopropyl-betaine, cocamidopropyl-betaine, linoleamidopropyl-betaine, myristamidopropyl-betaine, palmitamidopropyl-betaine, isostearamidopropyl-betaine, myristamidopropyl-dimethylamine, palmitamidopropyl-dimethylamine, isostearamidopropyl-dimethylamine, sodium methyl cocoyl, sodium methyl oleyl taurate, polyethylene glycol, polypropylene glycol, copolymers of ethylene and propylene glycol, etc., and any combination thereof. In some embodiments, the surfactant is polysorbate or poloxamer. In some embodiments, the surfactant is polysorbate 80, polysorbate 20, or poloxamer 188.

In some embodiments, the osmotic pressure regulator is a sugar (including monosaccharides, disaccharides, trisaccharides, polysaccharides, sugar alcohols, reducing sugars, non-reducing sugars, etc.), an amino acid (including arginine, glycine, cysteine, histidine, etc.), or a salt (sodium chloride, potassium chloride, calcium chloride, etc.). In some embodiments, the osmotic pressure regulator is a sugar selected from the group consisting of glucose, sucrose, trehalose, lactose, fructose, maltose, dextran, glycerin, erythritol, glycerol, arabitol, xylitol, sorbitol (also known as sorbol), mannitol, melibiose, melezitose, raffinose, mannotriose, stachyose, maltose, lactulose, maltulose, maltitol, lactitol, and iso-maltulose. In some embodiments, the osmotic pressure regulator is one or more selected from the group consisting of sucrose, trehalose, sorbitol, arginine, proline, glycine, and sodium chloride. In some embodiments, the osmotic pressure regulator is a non-reducing disaccharide; in some embodiments, the osmotic pressure regulator is trehalose and/or sucrose; in some embodiments, the osmotic pressure regulator is sucrose.

In some embodiments, the pharmaceutical composition comprising the anti-CD79b antibody-drug conjugate further comprises a solvent. Illustratively, the solvent is selected from the group consisting of, but is not limited to, non-toxic physiologically acceptable liquid carriers, such as normal saline, water for injection, and glucose solutions (e.g., a 5% injectable glucose solution and injectable glucose-sodium chloride solutions).

### <Rituximab>

In some embodiments, the immunotherapeutic agent is administered at a dose of about 1 mg/m² to about 1000 mg/m², e.g., about 1 mg/m², about 2.5 mg/m², about 5 mg/m², about 10 mg/m², about 12.5 mg/m², about 15 mg/m², about 17.5 mg/m², about 20 mg/m², about 25 mg/m², about 30 mg/m², about 35 mg/m², about 40 mg/m², about 45 mg/m², about 50 mg/m², about 60 mg/m², about 70 mg/m², about 75 mg/m², about 80 mg/m², about 90 mg/m², about 100 mg/m², about 125 mg/m², about 150 mg/m², about 175 mg/m², about 200 mg/m², about 225 mg/m², about 250 mg/m², about 275 mg/m², about 300 mg/m², about 325 mg/m², about 350 mg/m², about 375 mg/m², about 400 mg/m², about 425 mg/m², about 450 mg/m², about 475 mg/m², about 500 mg/m², about 600 mg/m², about 700 mg/m², about 750 mg/m², about 800 mg/m², about 900 mg/m², about 1000 mg/m², or any range between these point values.

In some specific embodiments, the immunotherapeutic agent is administered at a dose of about 375 mg/m².

In some embodiments, the immunotherapeutic agent is preferably administered at a frequency of once weekly, once every 2 weeks, once every 3 weeks, once every 4 weeks, once every 6 weeks, or once every 8 weeks. In some specific embodiments, the immunotherapeutic agent is administered at a frequency of once every 3 weeks.

In some embodiments, the administration regimen for rituximab is selected from the group consisting of any one of the following:
(b₁) rituximab is administered at a dose of about 1 mg/m² to about 1000 mg/m² and at a frequency of once weekly, once every 2 weeks, once every 3 weeks, once every 4 weeks, once every 6 weeks, or once every 8 weeks;
(b₂) rituximab is administered at a dose of about 10 mg/m² to about 1000 mg/m² and at a frequency of once weekly, once every 2 weeks, once every 3 weeks, once every 4 weeks, once every 6 weeks, or once every 8 weeks;
(b₃) rituximab is administered at a dose of about 50 mg/m² to about 1000 mg/m² and at a frequency of once weekly, once every 2 weeks, once every 3 weeks, once every 4 weeks, once every 6 weeks, or once every 8 weeks;
(b₄) rituximab is administered at a dose of about 50 mg/m² to about 500 mg/m² and at a frequency of once weekly, once every 2 weeks, once every 3 weeks, once every 4 weeks, once every 6 weeks, or once every 8 weeks;
(b₅) rituximab is administered at a dose of about 100 mg/m² to about 500 mg/m² and at a frequency of once weekly, once every 2 weeks, once every 3 weeks, once every 4 weeks, once every 6 weeks, or once every 8 weeks;
(b₆) rituximab is administered at a dose of about 325 mg/m², about 350 mg/m², about 375 mg/m², about 400 mg/m², or about 425 mg/m² and at a frequency of once weekly, once every 2 weeks, once every 3 weeks, once every 4 weeks, once every 6 weeks, or once every 8 weeks;
(b₇) rituximab is administered at a dose of about 325 mg/m², about 350 mg/m², about 375 mg/m², about 400 mg/m², or about 425 mg/m² and at a frequency of once every 3 weeks; and
(b₈) rituximab is administered at a dose of about 375 mg/m² and at a frequency of once every 3 weeks.

In some embodiments, in the administration regimen according to any one of the (b₁)-(b₆) described above, rituximab is administered at a frequency of once every 3 weeks.

### <Gemcitabine>

In some embodiments, gemcitabine is administered at a dose of about 5 mg/m² to about 2000 mg/m², e.g., about 5 mg/m², about 10 mg/m², about 12.5 mg/m², about 15 mg/m², about 17.5 mg/m², about 20 mg/m², about 25 mg/m², about 30 mg/m², about 35 mg/m², about 40 mg/m², about 45 mg/m², about 50 mg/m², about 60 mg/m², about 70 mg/m², about 75 mg/m², about 80 mg/m², about 90 mg/m², about 100 mg/m², about 125 mg/m², about 150 mg/m², about 175 mg/m², about 200 mg/m², about 225 mg/m², about 250 mg/m², about 275 mg/m², about 300 mg/m², about 325 mg/m², about 350 mg/m², about 375 mg/m², about 400 mg/m², about 425 mg/m², about 450 mg/m², about 475 mg/m², about 500 mg/m², about 600 mg/m², about 700 mg/m², about 750 mg/m², about 800 mg/m², about 900 mg/m², about 1000 mg/m², about 1100 mg/m², about 1125 mg/m², about 1150 mg/m², about 1175 mg/m², about 1200 mg/m², about 1225 mg/m², about 1250 mg/m², about 1275 mg/m², about 1300 mg/m², about 1325 mg/m², about 1350 mg/m², about 1375 mg/m², about 1400 mg/m², about 1425 mg/m², about 1450 mg/m², about 1475 mg/m², about 1500 mg/m², about 1600 mg/m², about 1700 mg/m², about 1750 mg/m², about 1800 mg/m², about 1900 mg/m², about 2000 mg/m², or any range between these point values.

In some embodiments, gemcitabine is administered at a dose of about 1000 mg/m².

In some embodiments, gemcitabine is administered at a frequency of once weekly, once every 2 weeks, once every 3 weeks, once every 4 weeks, once every 6 weeks, or once every 8 weeks. In some specific embodiments, gemcitabine is administered at a frequency of once every 3 weeks.

In some embodiments, gemcitabine is administered at a dose of about 5 mg/m² to about 2000 mg/m² and at a frequency of once every 3 weeks. In some specific embodiments, gemcitabine is administered at a dose of about 1000 mg/m² and at a frequency of once every 3 weeks.

### <Oxaliplatin>

In some embodiments, oxaliplatin is administered at a dose of about 1 mg/m² to about 500 mg/m², e.g., about 5 mg/m², about 10 mg/m², about 12.5 mg/m², about 15 mg/m², about 17.5 mg/m², about 20 mg/m², about 25 mg/m², about 30 mg/m², about 35 mg/m², about 40 mg/m², about 45 mg/m², about 50 mg/m², about 60 mg/m², about 70 mg/m², about 75 mg/m², about 80 mg/m², about 90 mg/m², about 100 mg/m², about 125 mg/m², about 150 mg/m², about 175 mg/m², about 200 mg/m², about 225 mg/m², about 250 mg/m², about 275 mg/m², about 300 mg/m², about 325 mg/m², about 350 mg/m², about 375 mg/m², about 400 mg/m², about 425 mg/m², about 450 mg/m², about 475 mg/m², about 500 mg/m², or any range between these point values.

In some embodiments, oxaliplatin is administered at a dose of about 100 mg/m².

In some embodiments, oxaliplatin is administered at a frequency of once weekly, once every 2 weeks, once every 3 weeks, once every 4 weeks, once every 6 weeks, or once every 8 weeks. In some specific embodiments, oxaliplatin is administered at a frequency of once every 3 weeks.

In some embodiments, oxaliplatin is administered at a dose of about 1 mg/m² to about 500 mg/m² and at a frequency of once every 3 weeks. In some specific embodiments, oxaliplatin is administered at a dose of about 100 mg/m² and at a frequency of once every 3 weeks.

### <Cyclophosphamide>

In some embodiments, cyclophosphamide is administered at a dose of about 1 mg/m² to about 1000 mg/m², e.g., about 1 mg/m², about 2.5 mg/m², about 5 mg/m², about 10 mg/m², about 12.5 mg/m², about 15 mg/m², about 17.5 mg/m², about 20 mg/m², about 25 mg/m², about 30 mg/m², about 35 mg/m², about 40 mg/m², about 45 mg/m², about 50 mg/m², about 60 mg/m², about 70 mg/m², about 75 mg/m², about 80 mg/m², about 90 mg/m², about 100 mg/m², about 125 mg/m², about 150 mg/m², about 175 mg/m², about 200 mg/m², about 225 mg/m², about 250 mg/m², about 275 mg/m², about 300 mg/m², about 325 mg/m², about 350 mg/m², about 375 mg/m², about 400 mg/m², about 425 mg/m², about 450 mg/m², about 475 mg/m², about 500 mg/m², about 600 mg/m², about 700 mg/m², about 750 mg/m², about 800 mg/m², about 900 mg/m², about 1000 mg/m², or any range between these point values.

In some embodiments, cyclophosphamide is administered at a dose of about 750 mg/m². In some embodiments, cyclophosphamide is administered at a frequency of once weekly, once every 2 weeks, once every 3 weeks, once every 4 weeks, once every 6 weeks, or once every 8 weeks. In some specific embodiments, cyclophosphamide is administered at a frequency of once every 3 weeks.

In some embodiments, cyclophosphamide is administered at a dose of about 1 mg/m² to about 1000 mg/m² and at a frequency of once every 3 weeks. In some specific embodiments, cyclophosphamide is administered at a dose of about 750 mg/m² and at a frequency of once every 3 weeks.

### <Anthracycline>

In some embodiments, the anthracycline is administered at a dose of about 1 mg/m² to about 500 mg/m², e.g., about 5 mg/m², about 10 mg/m², about 12.5 mg/m², about 15 mg/m², about 17.5 mg/m², about 20 mg/m², about 25 mg/m², about 30 mg/m², about 35 mg/m², about 40 mg/m², about 45 mg/m², about 50 mg/m², about 60 mg/m², about 70 mg/m², about 75 mg/m², about 80 mg/m², about 90 mg/m², about 100 mg/m², about 125 mg/m², about 150 mg/m², about 175 mg/m², about 200 mg/m², about 225 mg/m², about 250 mg/m², about 275 mg/m², about 300 mg/m², about 325 mg/m², about 350 mg/m², about 375 mg/m², about 400 mg/m², about 425 mg/m², about 450 mg/m², about 475 mg/m², about 500 mg/m², or any range between these point values.

In some embodiments, the anthracycline is administered at a dose of 30-40 mg/m², 30-50 mg/m², or 30-60 mg/m². In some embodiments, the anthracycline is doxorubicin, which is administered at a dose of 30-40 mg/m².

In some embodiments, the anthracycline is administered at a frequency of once weekly, once every 2 weeks, once every 3 weeks, once every 4 weeks, once every 6 weeks, or once every 8 weeks. In some specific embodiments, the anthracycline is administered at a frequency of once every 3 weeks.

In some embodiments, the anthracycline is administered at a dose of about 1 mg/m² to about 500 mg/m² and at a frequency of once every 3 weeks. In some specific embodiments, the anthracycline is administered at a dose of about 30-40 mg/m² and at a frequency of once every 3 weeks.

### <Vinca Alkaloid>

In some embodiments, the vinca alkaloid is administered at a dose of about 0.1 mg/m² to about 100 mg/m², e.g., about 0.1 mg/m², about 0.2 mg/m², about 0.3 mg/m², about 0.4 mg/m², about 0.5 mg/m², about 0.6 mg/m², about 0.7 mg/m², about 0.8 mg/m², about 0.9 mg/m², about 1.0 mg/m², about 1.1 mg/m², about 1.2 mg/m², about 1.3 mg/m², about 1.4 mg/m², about 1.5 mg/m², about 1.6 mg/m², about 1.7 mg/m², about 1.8 mg/m², about 1.9 mg/m², about 2.0 mg/m², about 2.1 mg/m², about 2.2 mg/m², about 2.3 mg/m², about 2.4 mg/m², about 2.5 mg/m², about 2.6 mg/m², about 2.7 mg/m², about 2.8 mg/m², about 2.9 mg/m², about 3.0 mg/m², about 3.25 mg/m², about 3.5 mg/m², about 3.75 mg/m², about 4.0 mg/m², about 4.25 mg/m², about 4.5 mg/m², about 4.75 mg/m², about 5.0 mg/m², about 5.25 mg/m², about 5.5 mg/m², about 5.75 mg/m², about 6.0 mg/m², about 6.25 mg/m², about 6.5 mg/m², about 6.75 mg/m², about 7.0 mg/m², about 7.25 mg/m², about 7.5 mg/m², about 7.75 mg/m², about 8.0 mg/m², about 9.0 mg/m², about 10 mg/m², about 12.5 mg/m², about 15 mg/m², about 17.5 mg/m², about 20 mg/m², about 25 mg/m², about 30 mg/m², about 35 mg/m², about 40 mg/m², about 45 mg/m², about 50 mg/m², about 60 mg/m², about 70 mg/m², about 75 mg/m², about 80 mg/m², about 90 mg/m², or about 100 mg/m².

In some embodiments, the vinca alkaloid is administered at a dose of about 1.4 mg/m². In some embodiments, the vinca alkaloid is administered at a dose of no more than 2 mg. In some embodiments, the vinca alkaloid is doxorubicin, which is administered at a dose of about 1.4 mg/m². In some embodiments, doxorubicin is administered at a dose of no more than 2 mg.

In some embodiments, the vinca alkaloid is administered at a frequency of once weekly, once every 2 weeks, once every 3 weeks, once every 4 weeks, once every 6 weeks, or once every 8 weeks. In some specific embodiments, the vinca alkaloid is administered at a frequency of once every 3 weeks.

In some embodiments, the anthracycline is administered at a dose of about 1 mg/m² to about 500 mg/m² and at a frequency of once every 3 weeks. In some specific embodiments, the anthracycline is administered at a dose of about 30-40 mg/m² and at a frequency of once every 3 weeks.

### <Prednisone>

In some embodiments, prednisone is administered at a dose of about 1 mg/time to about 1000 mg/time, e.g., about 5 mg/time, about 10 mg/time, about 12.5 mg/time, about 15 mg/time, about 17.5 mg/time, about 20 mg/time, about 25 mg/time, about 30 mg/time, about 35 mg/time, about 40 mg/time, about 45 mg/time, about 50 mg/time, about 60 mg/time, about 70 mg/time, about 75 mg/time, about 80 mg/time, about 90 mg/time, about 100 mg/time, about 125 mg/time, about 150 mg/time, about 175 mg/time, about 200 mg/time, about 225 mg/time, about 250 mg/time, about 275 mg/time, about 300 mg/time, about 325 mg/time, about 350 mg/time, about 375 mg/time, about 400 mg/time, about 425 mg/time, about 450 mg/time, about 475 mg/time, about 500 mg/time, about 510 mg/time, about 520 mg/time, about 530 mg/time, about 540 mg/time, about 550 mg/time, about 560 mg/time, about 580 mg/time, about 590 mg/time, about 600 mg/time, about 610 mg/time, about 620 mg/time, about 630 mg/time, about 640 mg/time, about 650 mg/time, about 660 mg/time, about 680 mg/time, about 690 mg/time, about 700 mg/time, about 710 mg/time, about 720 mg/time, about 730 mg/time, about 740 mg/time, about 750 mg/time, about 760 mg/time, about 780 mg/time, about 790 mg/time, about 800 mg/time, about 810 mg/time, about 820 mg/time, about 830 mg/time, about 840 mg/time, about 850 mg/time, about 860 mg/time, about 880 mg/time, about 890 mg/time, about 900 mg/time, about 910 mg/time, about 920 mg/time, about 930 mg/time, about 940 mg/time, about 950 mg/time, about 960 mg/time, about 980 mg/time, about 990 mg/time, or about 1000 mg/time.

In some embodiments, prednisone is administered at a dose of about 100 mg/time.

In some embodiments, prednisone is administered at a frequency of at least once weekly, at least once every 2 weeks, at least once every 3 weeks, at least once every 4 weeks, at least once every 6 weeks, or at least once every 8 weeks. In some embodiments, prednisone is administered at a frequency of at least once every 3 weeks. In some embodiments, prednisone is administered at a frequency of 3, 4, or 5 times every 3 weeks. In some embodiments, prednisone is administered at a frequency of 5 times every 3 weeks. In some embodiments, prednisone is administered at a dose of about 1 mg/time to about 1000 mg/time and at a frequency of 3, 4, or 5 times every 3 weeks. In some specific embodiments, prednisone is administered at a dose of about 100 mg/time and at a frequency of 5 times every 3 weeks.

### <Ifosfamide>

In some embodiments, ifosfamide is administered at a dose of about 0.1 mg/m² to about 100 mg/m², e.g., about 0.1 mg/m², about 0.5 mg/m², about 0.75 mg/m², about 1.0 mg/m², about 1.25 mg/m², about 1.5 mg/m², about 1.75 mg/m², about 2.0 mg/m², about 2.25 mg/m², about 2.5 mg/m², about 2.75 mg/m², about 3.0 mg/m², about 3.25 mg/m², about 3.5 mg/m², about 3.75 mg/m², about 4.0 mg/m², about 4.25 mg/m², about 4.5 mg/m², about 4.75 mg/m², about 5.0 mg/m², about 5.25 mg/m², about 5.5 mg/m², about 5.75 mg/m², about 6.0 mg/m², about 6.25 mg/m², about 6.5 mg/m², about 6.75 mg/m², about 7.0 mg/m², about 7.25 mg/m², about 7.5 mg/m², about 7.75 mg/m², about 8.0 mg/m², about 9.0 mg/m², about 10 mg/m², about 12.5 mg/m², about 15 mg/m², about 17.5 mg/m², about 20 mg/m², about 25 mg/m², about 30 mg/m², about 35 mg/m², about 40 mg/m², about 45 mg/m², about 50 mg/m², about 60 mg/m², about 70 mg/m², about 75 mg/m², about 80 mg/m², about 90 mg/m², or about 100 mg/m².

In some embodiments, ifosfamide is administered at a dose of about 5 mg/m².

In some embodiments, ifosfamide is administered at a frequency of once weekly, once every 2 weeks, once every 3 weeks, once every 4 weeks, once every 6 weeks, or once every 8 weeks. In some specific embodiments, ifosfamide is administered at a frequency of once every 3 weeks.

In some embodiments, ifosfamide is administered at a dose of about 0.1 mg/m² to about 100 mg/m² and at a frequency of once every 3 weeks. In some specific embodiments, ifosfamide is administered at a dose of about 5 mg/m² and at a frequency of once every 3 weeks.

### <Carboplatin>

In some embodiments, carboplatin is administered at a dose of about 1 mg to about 2000 mg, e.g., about 5 mg, about 10 mg, about 12.5 mg, about 15 mg, about 17.5 mg, about 20 mg, about 25 mg, about 30 mg, about 35 mg, about 40 mg, about 45 mg, about 50 mg, about 60 mg, about 70 mg, about 75 mg, about 80 mg, about 90 mg, about 100 mg, about 125 mg, about 150 mg, about 175 mg, about 200 mg, about 225 mg, about 250 mg, about 275 mg, about 300 mg, about 325 mg, about 350 mg, about 375 mg, about 400 mg, about 425 mg, about 450 mg, about 475 mg, about 500 mg, about 600 mg, about 700 mg, about 750 mg, about 800 mg, about 900 mg, about 1000 mg, about 1100 mg, about 1125 mg, about 1150 mg, about 1175 mg, about 1200 mg, about 1225 mg, about 1250 mg, about 1275 mg, about 1300 mg, about 1325 mg, about 1350 mg, about 1375 mg, about 1400 mg, about 1425 mg, about 1450 mg, about 1475 mg, about 1500 mg, about 1600 mg, about 1700 mg, about 1750 mg, about 1800 mg, about 1900 mg, about 2000 mg, or any range between these point values.

In some embodiments, carboplatin is administered at a dose of ≤800 mg, e.g., about 5 mg, about 10 mg, about 12.5 mg, about 15 mg, about 17.5 mg, about 20 mg, about 25 mg, about 30 mg, about 35 mg, about 40 mg, about 45 mg, about 50 mg, about 60 mg, about 70 mg, about 75 mg, about 80 mg, about 90 mg, about 100 mg, about 125 mg, about 150 mg, about 175 mg, about 200 mg, about 225 mg, about 250 mg, about 275 mg, about 300 mg, about 325 mg, about 350 mg, about 375 mg, about 400 mg, about 425 mg, about 450 mg, about 475 mg, about 500 mg, about 510 mg, about 520 mg, about 530 mg, about 540 mg, about 550 mg, about 560 mg, about 580 mg, about 590 mg, about 600 mg, about 610 mg, about 620 mg, about 630 mg, about 640 mg, about 650 mg, about 660 mg, about 680 mg, about 690 mg, about 700 mg, about 710 mg, about 720 mg, about 730 mg, about 740 mg, about 750 mg, about 760 mg, about 780 mg, about 790 mg, or about 800 mg.

In some embodiments, carboplatin is administered at a frequency of once weekly, once every 2 weeks, once every 3 weeks, once every 4 weeks, once every 6 weeks, or once every 8 weeks. In some specific embodiments, carboplatin is administered at a frequency of once every 3 weeks.

In some embodiments, carboplatin is administered at a dose of about 1 mg to about 2000 mg and at a frequency of once every 3 weeks. In some specific embodiments, carboplatin is administered at a dose of ≤800 mg and at a frequency of once every 3 weeks.

### <Etoposide Phosphate>

In some embodiments, etoposide phosphate is administered at a dose of about 1 mg/m²/time to about 500 mg/m²/time, e.g., about 5 mg/m²/time, about 10 mg/m²/time, about 12.5 mg/m²/time, about 15 mg/m²/time, about 17.5 mg/m²/time, about 20 mg/m²/time, about 25 mg/m²/time, about 30 mg/m²/time, about 35 mg/m²/time, about 40 mg/m²/time, about 45 mg/m²/time, about 50 mg/m²/time, about 60 mg/m²/time, about 70 mg/m²/time, about 75 mg/m²/time, about 80 mg/m²/time, about 90 mg/m²/time, about 100 mg/m²/time, about 125 mg/m²/time, about 150 mg/m²/time, about 175 mg/m²/time, about 200 mg/m²/time, about 225 mg/m²/time, about 250 mg/m²/time, about 275 mg/m²/time, about 300 mg/m²/time, about 325 mg/m²/time, about 350 mg/m²/time, about 375 mg/m²/time, about 400 mg/m²/time, about 425 mg/m²/time, about 450 mg/m²/time, about 475 mg/m²/time, about 500 mg/m²/time, or any range between these point values.

In some embodiments, etoposide phosphate is administered at a dose of 30-40 mg/m², 30-50 mg/m², or 30-60 mg/m². In some embodiments, etoposide phosphate is doxorubicin, which is administered at a dose of 30-40 mg/m².

In some embodiments, etoposide phosphate is administered at a frequency of at least once weekly, at least once every 2 weeks, at least once every 3 weeks, at least once every 4 weeks, at least once every 6 weeks, or at least once every 8 weeks. In some embodiments, etoposide phosphate is administered at a frequency of at least once every 3 weeks. In some embodiments, etoposide phosphate is administered at a frequency of 3, 4, or 5 times every 3 weeks. In some embodiments, etoposide phosphate is administered at a frequency of 3 times every 3 weeks.

In some embodiments, etoposide phosphate is administered at a dose of about 1 mg/m²/time to about 500 mg/m²/time and at a frequency of 2, 3, or 4 times every 3 weeks. In some specific embodiments, etoposide phosphate is administered at a dose of about 100 mg/m²/time and at a frequency of 3 times every 3 weeks.

In some embodiments, provided is any one of the aforementioned methods or uses, wherein a subject to which the anti-CD79b antibody-drug conjugate is administered experiences a reduced clinical adverse reaction. In some embodiments, the clinical adverse reaction is peripheral neurotoxicity. In some specific embodiments, compared to a subject to which polatuzumab vedotin is administered, a subject to which the anti-CD79b antibody-drug conjugate in the present disclosure is administered experiences a reduced clinical adverse reaction. In some specific embodiments, compared to a subject to which polatuzumab vedotin is administered, a subject to which the anti-CD79b antibody-drug conjugate in the present disclosure is administered experiences reduced peripheral neurotoxicity.

In some embodiments, the anti-CD79b antibody-drug conjugate in the present disclosure, when administered to a patient with B-cell lymphoma alone or in combination with an immunotherapeutic agent or a chemotherapeutic agent, enables the patient to achieve a sustained response, exhibiting strong anti-tumor activity. Moreover, the monotherapy or combination therapy regimen in the present disclosure has low neurotoxicity and can improve the quality of life of patients, providing a safe and effective clinical treatment regimen for patients with B-cell lymphoma.

### Terminology

In order to facilitate the understanding of the present disclosure, certain technical and scientific terms are specifically defined below. Unless otherwise specifically defined herein, all other technical and scientific terms used herein have the meanings generally understood by those of ordinary skill in the art to which the present disclosure pertains. Unless the context clearly requires otherwise, throughout the description and the claims, the words "comprise", "have", "include", etc., are to be understood in an inclusive sense as opposed to an exclusive or exhaustive sense, that is to say, in the sense of "including but not limited to".

"Optional" or "optionally" means that the event or circumstance subsequently described may, but does not necessarily, occur; this description includes the instance where the event or circumstance occurs or does not occur.

"About" or "approximately" means that the numerical value is within an acceptable margin of error for the specific value determined by those of ordinary skill in the art, and the numerical value depends in part on how the value is measured or determined (i.e., the limitations of the measurement system). For example, "about" may mean a standard deviation within 1 or greater than 1. Alternatively, "about" or "substantially comprise" may mean a range of at most 20%, e.g., a variation of between 1% and 15%, between 1% and 10%, between 1% and 5%, between 0.5% and 5%, or between 0.5% and 1%. In the present disclosure, every instance where a number or numerical range is preceded by the term "about" also includes embodiments of the given number. Unless otherwise specified, when a specific value is provided in the present application and claims, the meaning of "about" or "substantially comprise" should be assumed to be within an acceptable margin of error for that specific value.

The term "and/or", e.g., "X and/or Y", should be understood to mean "X and Y" or "X or Y" and should be used to provide explicit support for both meanings or either meaning. In some embodiments, the present disclosure provides the following definitions:
An "antibody-drug conjugate" (ADC) is formed by linking an antibody or an antibody fragment to a cytotoxin with biological activity or a small-molecule drug with cell killing activity by a stable chemical linker compound; the binding specificity of the antibody to a tumor cell-specific or highly expressed antigen and the high efficiency of the cytotoxin are combined to prevent toxic and side effects on normal cells. Compared to conventional chemotherapeutic drugs, antibody-drug conjugates can bind to tumor cells precisely and have less impact on normal cells.

If an antibody-drug conjugate shows no significant chemical change, then the antibody "retains its chemical stability" in a pharmaceutical formulation. Chemical stability can be assessed by detecting and quantifying proteins in chemically altered forms. Degradation processes that often change the chemical structure of a protein include hydrolysis or clipping (evaluated by methods such as size exclusion chromatography and CE-SDS), oxidation (evaluated by methods such as peptide mapping combined with mass spectrometry or MALDI/TOF/MS), deamidation (evaluated by methods such as ionexchange chromatography, capillary isoelectric focusing, peptide mapping, and isoaspartic acid measurement), and isomerization (evaluated by isoaspartic acid content measurement, peptide mapping, etc.).

An antibody-drug conjugate "retains its biological activity" in a pharmaceutical formulation if the biological activity of the antibody-drug conjugate at a given time is within a predetermined range of the biological activity exhibited at the time the pharmaceutical formulation was prepared.

The three-letter and single-letter codes for amino acids used in the present disclosure are described in J. Biol. Chem., 243, p3558 (1968).

As used herein, the term "antibody" is used in the broadest sense and encompasses a variety of antibody structures, including but not limited to monoclonal antibodies, polyclonal antibodies, monospecific antibodies, multispecific antibodies (e.g., bispecific antibodies), full-length antibodies, and antibody fragments (or antigen-binding fragments, or antigen-binding moieties), so long as they exhibit the desired antigen-binding activity. An antibody may refer to an immunoglobulin, which is a four-peptide-chain structure formed by linking two identical heavy chains and two identical light chains by interchain disulfide bonds. The heavy chain constant regions of immunoglobulins differ in amino acid composition and arrangement; therefore, they also differ in antigenicity. Accordingly, immunoglobulins can be classified into five classes, also referred to as isotypes of immunoglobulins, namely IgM, IgD, IgG, IgA, and IgE, and their corresponding heavy chains are a µ chain, a δ chain, a γ chain, an α chain, and an ε chain, respectively. Igs of the same class can be classified into different subclasses based on the amino acid composition of their hinge regions and the number and positions of heavy chain disulfide bonds; for example, IgG can be divided into IgG1, IgG2, IgG3, and IgG4. Light chains are classified into κ or λ chains based on their constant regions. Each of the five Ig classes may have a κ chain or λ chain. In the antibody heavy and light chains, the sequences of about 110 amino acids near the N-terminus vary considerably and thus are referred to as variable regions (V regions); the remaining amino acid sequences near the C-terminus are relatively stable and thus are referred to as constant regions (C regions). The variable regions comprise 3 hypervariable regions (CDRs) and 4 framework regions (FRs) with relatively conservative sequences. The 3 hypervariable regions determine the specificity of the antibody and thus are also known as complementarity-determining regions (CDRs). Each of the light chain variable regions (VLs) and the heavy chain variable regions (VHs) consists of 3 CDRs and 4 FRs arranged from the amino-terminus to the carboxyl-terminus as follows: FR1, CDR1, FR2, CDR2, FR3, CDR3, and FR4. The 3 CDRs of the light chain refer to LCDR1, LCDR2, and LCDR3, and the 3 CDRs of the heavy chain refer to HCDR1, HCDR2, and HCDR3.

For the determination or definition of CDRs, the deterministic depiction of CDRs and identification of residues comprising binding sites of an antibody can be accomplished by resolving the structure of the antibody and/or resolving the structure of the antibody-ligand complex. This can be accomplished by any one of a variety of techniques known to those skilled in the art, such as X-ray crystallography. A variety of analysis methods can be used to identify CDRs, including but not limited to the Kabat numbering scheme, the Chothia numbering scheme, the AbM numbering scheme, the IMGT numbering scheme, the contact definition, and the conformational definition.

The amino acid sequence boundaries of the CDRs can be determined by a variety of well-known schemes, for example, the "Kabat" numbering scheme (see Kabat et al. (1991), "Sequences of Proteins of Immunological Interest", 5th Edition, Public Health Service, National Institutes of Health, Bethesda, MD), the "Chothia" numbering scheme, the "ABM" numbering scheme, the "contact" numbering scheme (see Martin, ACR. Protein Sequence and Structure Analysis of Antibody Variable Domains[J]. 2001), the ImMunoGenTics (IMGT) numbering scheme (Lefranc, M.P. et al., Dev. Comp. Immunol., 27, 55-77 (2003); Front Immunol. 2018 Oct 16; 9: 2278), etc.

The term "antigen-binding fragment" or "functional fragment" or "antigen-binding moiety" refers to one or more fragments of an intact antibody that retain the ability to specifically bind to an antigen. It has been shown that a fragment of a full-length antibody can be used to achieve the antigen-binding function of the antibody. Illustratively, examples of binding fragments encompassed by the term "antigen-binding fragment" include: (i) a Fab fragment, a monovalent fragment consisting of VL, VH, CL, and CH1 domains; (ii) a F(ab')₂ fragment, a bivalent fragment comprising two Fab fragments linked by a disulfide bridge in the hinge region; (iii) an Fd fragment consisting of VH and CH1 domains; (iv) an Fv fragment consisting of the VH and VL domains of a single arm of an antibody; (v) a dsFv, a stable antigen-binding fragment formed by linking a VH and a VL by an interchain disulfide bond; (vi) an scFv; (vii) a diabody, a bispecific antibody, and a multispecific antibody comprising fragments such as an scFv, a dsFv, and a Fab.

The terms "specific binding", "selective binding", "selectively bind to", and "specifically bind to" refer to the binding of an antibody to an epitope on a predetermined antigen. Generally, the antibody binds with an affinity (KD) of less than about 10⁻⁸ M, e.g., less than about 10⁻⁹ M, 10⁻¹⁰ M, or 10⁻¹¹ M or less.

The term "KD" refers to the dissociation equilibrium constant for a specific antibody-antigen interaction. Generally, the antibody of the present disclosure binds to IL-5 with a dissociation equilibrium constant (KD) of less than about 10⁻⁷ M, e.g., less than about 10⁻⁸ M, 10⁻⁹ M, or 10⁻¹⁰ M or less, for example, as determined in a BIACORE instrument using surface plasmon resonance (SPR) technology.

"Homology" refers to the sequence similarity between two polynucleotide sequences or between two polypeptides. When positions in two sequences being compared are occupied by identical bases or amino acid monomer subunits, for example, if a position is occupied by adenine in both DNA molecules, the molecules are homologous at that position. The homology percentage between two sequences is a function of the number of matching or homologous positions shared by the two sequences divided by the number of positions compared × 100%. For example, in the optimal alignment of sequences, if 6 out of 10 positions of two sequences are matched or homologous, the two sequences are 60% homologous, and if 95 out of 100 positions of two sequences are matched or homologous, the two sequences are 95% homologous. Generally, two sequences, when aligned, are compared to give the maximum percent homology. For example, the comparison may be made using a BLAST algorithm, wherein parameters are selected for the algorithm to give the maximum match between the reference sequences over the entire length of each sequence. The following references relate to BLAST algorithms often used for sequence analysis: BLAST algorithms: Altschul, S.F. et al., (1990) J. Mol. Biol., 215: 403-410; Gish, W., et al., (1993) Nature Genet., 3: 266-272; Madden, T.L. et al., (1996) Meth. Enzymol., 266: 131-141; Altschul, S.F. et al., (1997) Nucleic Acids Res., 25: 3389-3402; and Zhang, J. et al., (1997) Genome Res., 7: 649-656. Other conventional BLAST algorithms, such as the one provided by NCBI BLAST, are also well known to those skilled in the art.

"Administer" and "treat", when applied to animals, humans, experimental subjects, cells, tissues, organs, or biological fluids, refer to contacting an exogenous drug, a therapeutic agent, a diagnostic agent, or a composition with the animals, humans, subjects, cells, tissues, organs, or biological fluids. "Administer" and "treat" can refer to, for example, therapeutic, pharmacokinetic, diagnostic, research, and experimental methods. Treatment of cells comprises contact between a reagent and cells, and contact between the reagent and a fluid, wherein the fluid is in contact with the cells. "Administer" and "treat" also mean treating, for example, a cell with a reagent, diagnostic, or binding composition or with another cell *in vitro* and *ex vivo*. "Treat", when applied to humans, veterinary, or research subjects, refers to therapeutic treatment, prophylactic or preventive measures, and research and diagnostic applications.

"Treatment" refers to administering a therapeutic agent, such as a composition comprising any one of the conjugation compounds of the present disclosure, either internally or externally, to a patient with one or more disease symptoms on which the therapeutic agent is known to have a therapeutic effect. Generally, the therapeutic agent is administered in an amount effective to alleviate one or more disease symptoms in the patient or population being treated to induce regression of such symptoms or to inhibit the development of such symptoms to any clinically measurable degree. The amount of the therapeutic agent effective to alleviate any specific disease symptom (also known as a "therapeutically effective amount") may vary depending on a variety of factors, such as the disease state, age, and body weight of the patient, and the ability of the drug to produce a desired therapeutic effect in the patient. Whether a disease symptom has been palliated can be evaluated by any clinical test method commonly used by doctors or other health care professionals to evaluate the severity or progression of the symptom. Although an embodiment of the present disclosure (for example, a treatment method or a product) may not be effective in alleviating every disease symptom of interest, it should palliate the disease symptoms of interest in a statistically significant number of patients, as determined by any statistical test method known in the art, such as a Student t-test, a chi-square test, a Mann and Whitney's U test, a Kruskal-Wallis test (H test), a Jonckheere-Terpstra test, and a Wilcoxon test.

"Effective amount" comprises an amount sufficient to ameliorate or prevent a symptom or sign of a medical disease. The effective amount also means an amount sufficient to allow or facilitate diagnosis. The effective amount for a particular patient or veterinary subject may vary depending on factors such as the disorder to be treated, the general health of the patient, the method, route, and dose of administration, and the severity of side effects. The effective amount may be the maximum dose or administration regimen to avoid significant side effects or toxic effects.

The term "subject" or "patient" means a mammal, particularly a primate, and particularly a human.

The "combination" described in the present disclosure is a mode of administration and refers to the administration of at least one dose of an anti-CD79b antibody-drug conjugate and at least one dose of an immunotherapeutic agent and/or a chemotherapeutic agent over a certain period of time, wherein both drugs exhibit pharmacological effects. The period of time may be within one administration cycle, preferably within 4 weeks, within 3 weeks, within 2 weeks, within 1 week, within 24 h, or within 12 h. The anti-CD79b antibody-drug conjugate and drugs of other classes may be administered simultaneously or sequentially. Such periods of time include treatment in which the anti-CD79b antibody-drug conjugate and drugs of other classes are administered via the same route of administration or different routes of administration. The combination administration described in the present disclosure is selected from the group consisting of simultaneous administration, independent formulation and co-administration, and independent formulation and sequential administration.

The "n" in the anti-CD79b antibody-drug conjugate of the present disclosure refers to the average number of cytotoxic drugs loaded on each antibody or an antigen-binding fragment thereof in the antibody-drug conjugate molecule, and it may also be expressed as the ratio of the number of drugs to the number of antibodies, which is the average number of drugs per ADC molecule after a coupling reaction as determined by hydrophobic interaction chromatography (HIC) or mass spectrometry.

"Pharmaceutical composition" refers to a mixture comprising one or more of the compounds or the physiologically/pharmaceutically acceptable salts or pro-drugs thereof described herein and other chemical components, and the other components are, for example, physiologically/pharmaceutically acceptable carriers and excipients. The pharmaceutical composition is intended to promote administration to an organism and facilitate the absorption of the active ingredient so that it can exert its biological activity.

### DETAILED DESCRIPTION

The present disclosure is further described below with reference to examples, which, however, are not intended to limit the scope of the present disclosure. In the examples of the present disclosure, experimental methods in which specific conditions are not indicated were generally carried out under conventional conditions, for example, as described in Antibodies: A Laboratory Manual and Molecular Cloning: A Laboratory Manual by Cold Spring Harbor Laboratory, or in accordance with the conditions recommended by the manufacturers of the starting materials or commercial products. Reagents whose specific sources are not indicated were commercially available conventional reagents.

### Example 1. Preparation of Anti-CD79b Antibody-Drug Conjugate

The anti-CD79b antibody-drug conjugate was the ADC-6 prepared in Example 3-3 in WO2023143347A1, which is incorporated herein by reference in its entirety. Its structure is shown below:

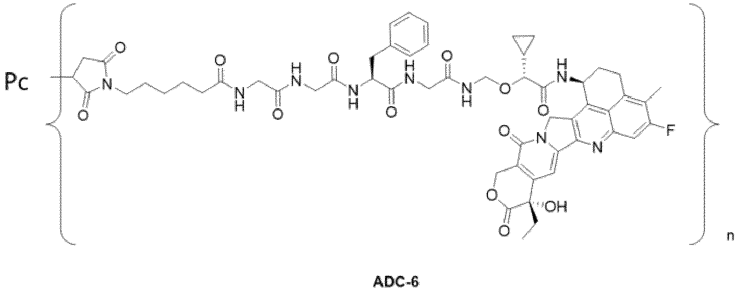

The mean value calculated by RP-HPLC: n = 3.46.

The anti-CD79b antibody was derived from the antibody hAb015-10 in WO2022022508A1, which is incorporated herein by reference in its entirety. Its CDR sequences are shown in Table 1. The full-length heavy chain of antibody hAb015-10 is set forth in SEQ ID NO: 12, and its full-length light chain is set forth in SEQ ID NO: 13.

### Example 2. Phase I Clinical Study on Safety, Tolerability, Pharmacokinetics, and Efficacy of ADC-6 Monotherapy for Injection in Patients with B-Cell Lymphoma

### 1. Investigational drug

1) The ADC-6 prepared in Example 1. Sterile powder for injection, strength: 70 mg/vial.

### 2. Enrolled subjects

1) Age ≥ 18 years, any sex.
2) ECOG performance status of 0 or 1.
3) Expected survival ≥ 12 weeks.
4) Cytologically or histologically confirmed B-cell lymphoma.
5) Has received ≥1 prior line of standard anti-tumor therapy, and did not achieve a response after the most recent treatment or experienced disease progression after treatment (for FL, a treatment indication must be present). If standard therapy contained CD20-targeted therapy, all must have received adequate prior therapy containing rituximab or another CD20-targeted drug or experienced disease progression or intolerance during treatment. In addition, patients with MCL, CLL/SLL, or WM have received at least two prior lines of therapy, including a BTK inhibitor.
6) Subjects with diffuse large B lymphoma in the efficacy expansion phase must have received ≥2 prior lines of systemic anti-tumor therapy.
7) Presence of a measurable lesion (for lymphoma, the long diameter of a lymph node lesion should be >1.5 cm, or the long diameter of an extranodal lesion should be >1.0 cm, and it is not mandatory in monotherapy dose escalation studies).

### 3. Clinical protocol

The starting dose of ADC-6 was 0.1 mg/kg, and 11 dose groups (0.1 mg/kg, 0.3 mg/kg, 0.6 mg/kg, 1.2 mg/kg, 1.8 mg/kg, 2.7 mg/kg, 3.6 mg/kg, 4.8 mg/kg, 6.4 mg/kg, 8.0 mg/kg, and 10 mg/kg) were preset. The drug was administered via intravenous drip infusion once every 3 weeks (Q3W), with 21 days as one treatment cycle. The first cycle (21 days) was used as the DLT observation period.

### 4. Results

4.1. The main adverse events (AEs) were hematologic toxicities, laboratory test value abnormalities, and digestive tract reactions, mostly grade 1-2. Treatment-emergent adverse events (TEAEs) occurring in ≥20% of patients were: decreased neutrophil count, decreased leukocyte count, nausea, vomiting, decreased platelet count, elevated blood bilirubin, elevated aspartate aminotransferase, COVID-19, and elevated alanine aminotransferase. Grade ≥3 TEAEs occurring in ≥2 subjects included: decreased neutrophil count (34.7%), decreased leukocyte count (20.4%), COVID-19 pneumonia (12.2%), progressive tumor (12.2%), infectious pneumonia (10.2%), anemia (10.2%), decreased lymphocyte count (8.2%), decreased platelet count (8.2%), and bacterial pneumonia (6.1%). Among 27 efficacy-evaluable DLBCL subjects (7 at 1.8 mg/kg, 7 at 2.7 mg/kg, and 9 at 3.6 mg/kg; ≥1 prior line of standard therapy), the overall ORR (objective response rate) was 59.3% (16/27), and the CRR (complete response rate) was 18.5% (5/27).

It can be seen from the above results that in the Phase I study, ADC-6 exhibited preliminary efficacy against DLBCL and a favorable safety profile, and there were no reports of peripheral neurotoxicity, indicating clinical potential for further developing combination therapy regimens.

### Example 3. Phase I Clinical Study on Safety, Tolerability, Pharmacokinetics, and Efficacy of ADC-6 Monotherapy for Injection in Patients with B-Cell Lymphoma 1. Study method

The subjects were patients with histologically confirmed B-NHL who had received ≥1 prior session of anti-cancer therapy but achieved a poor response or experienced disease progression. The dose escalation (D-ESC) part began with accelerated titration at doses of 0.1 and 0.3 mg/kg and then switched to an i3+3 regimen with dose escalation starting at 0.6 mg/kg (Q3W, intravenous injection).

In the dose expansion (D-EXP) part, more patients were enrolled at selected doses (a total of about 10-12 patients per dosage level). The primary objectives were to assess the safety and tolerability of **ADC-6** and to determine the RP2D.

### 2. Results

2.1. As of November 7, 2023, a total of 49 patients had been enrolled (1 in the 0.1 mg/kg dose group, 1 in the 0.3 mg/kg dose group, 7 in the 0.6 mg/kg dose group, 3 in the 1.2 mg/kg dose group, 13 in the 1.8 mg/kg dose group, 13 in the 2.7 mg/kg dose group, and 11 in the 3.6 mg/kg dose group), including 33 patients with diffuse large B-cell lymphoma (DLBCL), 12 patients with follicular lymphoma (FL), and 4 patients with marginal zone lymphoma (MZL). The median number of prior treatments received by the patients was 2 (ranging from 1 to 9). Overall, 20 patients (40.8%) experienced grade ≥3 treatment-related adverse events, with decreased neutrophil count being the most common adverse event (≥20%).

Among 41 patients with baseline target lesions who underwent at least one post-baseline assessment, the ORR was 56.1% (95% CI, 39.8-71.5), and the 6-month DoR rate was 62.7% (95% CI, 26.7-84.8). In terms of subtype, the ORR was 51.9% among DLBCL patients (57.1% in the 2.7 mg/kg group and 77.8% in the 3.6 mg/kg group), 63.6% among FL patients, and 66.7% among MZL patients (Table). In the 1.8-3.6 mg/kg dose groups, the mean half-life of the conjugated antibody was 4.7-5.3 days, and the volume of distribution approximated human plasma volume. The exposure level of free toxins in plasma was relatively low, the toxin/conjugate antibody molar ratio was less than 1%, and no accumulation was observed.

**Table 2. The effectiveness of ADC-6 in treating cancer patients**

| | **DLBCL (n=27)** | **FL (n=11)** | **MZL (n=3)** | **All (n=41)** |
|---|---|---|---|---|
| Best overall response of tumor | | | | |
| Complete response (CR) | 4 (14.8%) | 0 | 0 | 4 (9.8) |
| Partial response (PR) | 10 (37.0%) | 7 (63.6) | 2 (66.7) | 19 (46.3) |
| Stable disease (SD) | 5 (18.5%) | 2 (18.2) | 0 | 7 (17.1) |
| Progressive disease (PD) | 8 (29.6%) | 2 (18.2) | 1 (33.3) | 11 (26.8) |
| Objective response rate (ORR) | 51.9% (32.0-71.3) | 63.6% (30.8-89.1) | 66.7% (9.4-99.2) | 56.1% (39.8-71.5) |
| Disease control rate (DCR) | 70.4% (49.8-86.3) | 81.8% (48.2-97.7) | 66.7% (9.4-99.2) | 73.2% (57.1-85.8) |
| 6-month duration of response rate (6-mo DoR rate) | 65.6% (15.7-90.9) | 33.3% (0.90-77.4) | 100.0 (100.0-100.0) | 62.7% (26.7-84.8) |

| | | | | |
|---|---|---|---|---|
| Data are shown as n (%) or % (95% CI). | | | | |

2.2. As of November 19, 2024, 33 patients (67.3%) had experienced grade ≥3 TEAEs, with decreased neutrophil count being the most common adverse event (≥20%). Among 49 patients (FAS), ADC-6 monotherapy resulted in an ORR of 51.0% (95% CI: 36.34, 65.58), a CRR of 10.2% (95% CI: 3.40, 22.23), an mPFS of 6.7 months [3.02, NR], and an mDOR of NR [5.19, NR]. Among 33 patients with relapsed or refractory DLBCL (FAS), the ORR was 48.5% (95% CI: 30.80, 66.46), the CRR was 15.2% (95% CI: 5.11, 31.90), the mPFS was 4.3 months [1.41, NR], and the mDOR was 10.4 months [4.21, NR]. In the 1.8-3.6 mg/kg dose groups, the mean half-life of the conjugated antibody was 4.7-5.3 days, and the volume of distribution approximated human plasma volume. The exposure level of free toxins in plasma was relatively low, the toxin/conjugate antibody molar ratio was less than 1%, and no accumulation was observed.

**Table 3. The effectiveness of ADC-6 in treating cancer patients**

| | Total | DLBCL |
|---|---|---|
| | (N=49) | (N=33) |
| | n(%) | n(%) |
| Best overall response (BOR) | | |
| Complete response (CR), n (%) | 5 (10.2%) | 5 (15.2%) |
| Partial response (PR), n (%) | 20 (40.8%) | 11 (33.3%) |
| Stable disease (SD), n (%) | 7 (14.3%) | 4 (12.1%) |
| Progressive disease (PD), n (%) | 11 (22.4%) | 8 (24.2%) |
| Not evaluable (NE), n (%) | 6 (12.2%) | 5 (15.2%) |
| Objective response rate (ORR = CR + PR), n (%) | 25 (51.0%) | 16 (48.5%) |
| 95% Exact CI^{[1]} | [36.34, 65.58] | [30.80, 66.46] |
| Median duration of objective response estimated by Kaplan-Meier method (months, 95% CI^{[2]}) | NR [5.19, NR] | 10.4 [4.21, NR] |
| Median progression-free survival estimated by Kaplan-Meier method (months, 95% CI^{[2]}) | 6.7 [3.02, NR] | 4.3 [1.41, NR] |

| | | |
|---|---|---|
| [1] The Clopper-Pearson method was used. [2] Estimated using the Brookmeyer and Crowley method. Data are shown as n (%) or % (95% CI). | | |

### Example 4. Phase IB/II Study of Combination Therapy with ADC-6 for Injection in B-Cell Lymphoma

### 1. Investigational drugs

- The ADC-6 prepared in Example 1.
- Injectable rituximab solution.
- Gemcitabine hydrochloride for injection.
- Oxaliplatin for injection.
- Ifosfamide for injection
- Carboplatin for injection
- Etoposide phosphate soft capsules
- Cyclophosphamide for injection
- Injectable doxorubicin liposome solution (or another anthracycline selected by the investigator)
- Vincristine sulfate for injection (or another vinca alkaloid selected by the investigator)
- Prednisone acetate tablets

### 2. Enrolled subjects

2.1. Subjects must meet all of the following criteria to be eligible for this study:
1. Confirmed diagnosis of diffuse large B-cell lymphoma (DLBCL), including *de novo* DLBCL and indolent lymphoma-transformed DLBCL, according to the 2016 revision of the WHO classification of lymphoid neoplasms.
2. The prior systemic anti-tumor therapy that the subject has received must meet the following requirements:
   - Subjects in the relapsed or refractory cohorts in the Phase Ib dose escalation and Phase Ib dose expansion phases and Phase II subjects must have received ≥1 prior line of systemic anti-tumor therapy, and did not achieve a response after the last line of therapy or experienced disease progression after treatment:
      relapsed: achieved a response during treatment, and relapsed 6 months or more after completing treatment;
      refractory: achieved no significant response during treatment, or relapsed within 6 months of completing treatment.
   - Subjects in the Phase Ib treatment-naive cohort have not received prior lymphoma-related systemic anti-tumor therapy.
3. Presence of a measurable lesion (for lymphoma, the long diameter of a lymph node lesion should be >1.5 cm, or the long diameter of an extranodal lesion should be >1.0 cm).
4. Expected survival of at least 3 months.
5. Age ≥ 18 years.
6. Eastern Cooperative Oncology Group (ECOG) performance status of 0-1.

### 2.2. Subjects will be excluded from this study if they meet any one of the following criteria:

Subjects receiving combination therapy with the R-Gemox regimen in the Phase Ib dose escalation/dose expansion phase and Phase II cohort 1 subjects have received prior treatment with gemcitabine. Subjects receiving combination therapy with the R-ICE/R-IC regimen in the Phase Ib dose escalation/dose expansion phase and Phase II cohort 2a/2b subjects have received prior treatment with ifosfamide.

### 3. Administration regimen

### 3.1. Dose escalation/expansion phase (Phase Ib):

3.1.1. Dose escalation phase: ADC-6 will be administered using a dose escalation design, and the immunochemotherapy regimen will be given at a fixed dose. The investigator will select either the R-Gemox regimen (for subjects who have not received prior treatment with gemcitabine) or the R-ICE/R-IC regimen (for subjects who have not received prior treatment with ifosfamide) based on the subject's prior treatment. The starting dose of ADC-6 will be 2.7 mg/kg, and at least 2 dose groups (2.7 mg/kg and 3.6 mg/kg) will be preset. Additional dose groups may be added based on the dose exploration results from ADC-6 monotherapy and combination therapy.

### 3.1.2. Dose expansion contains a relapsed or refractory cohort and a treatment-naive cohort.

Relapsed or refractory cohort: ADC-6 treatment will continue until disease progression, the subject's request for withdrawal from the study, or intolerable toxicity, whichever occurs first. Immunochemotherapy will continue until disease progression, the subject's request for withdrawal from the study, intolerable toxicity, completion of 6-8 treatment cycles (R-Gemox regimen), or completion of 4-6 treatment cycles (R-ICE/RIC regimen), whichever occurs first.

Treatment-naive cohort: The dose for this cohort will be determined based on the actual dose exploration results from the relapsed or refractory cohort. Subjects who have not received prior lymphoma-related systemic anti-tumor therapy will be enrolled. Subjects will receive ADC-6 plus 6 cycles of R-CHOP + 2 additional doses of R.

### 3.2. Efficacy expansion phase (Phase II):

Cohort 1: Subjects with relapsed or refractory DLBCL who have received ≥1 prior line of systemic anti-tumor therapy and have not received prior treatment with gemcitabine will be enrolled. Subjects will receive ADC-6 plus 6-8 cycles of treatment with R-GemOx. Cohort 2a/2b: Subjects with relapsed or refractory DLBCL who have received ≥1 prior line of systemic anti-tumor therapy and have not received prior treatment with ifosfamide will be enrolled. Subjects will receive ADC-6 plus 4-6 cycles of treatment with R-ICE (cohort 2a) or R-IC (cohort 2b).

The total dose of ADC-6 will be calculated based on the subject's body weight measured before each dose, and administration will be performed via intravenous drip infusion once every 3 weeks (Q3W), with every 3 weeks as one treatment cycle. Infusion requirements for other drugs should follow their respective package inserts and standard clinical practice. The administration regimens are specifically shown in Tables 3-6 below:

**Table 4. An administration schedule for combination therapy with the R-Gemox regimen in the Phase Ib dose escalation/expansion phase and Phase II cohort 1**

| | D1 | D2 |
|---|---|---|
| ADC-6 | | √ |
| Rituximab 375 mg/m² | √ | |
| Gemcitabine 1000 mg/m² | | √ |
| Oxaliplatin 100 mg/m² | | √ |

**Table 5. An administration schedule for the Phase Ib treatment-naive cohort**

| | D1 | D2 | D3 | D4 | D5 | D6 |
|---|---|---|---|---|---|---|
| ADC-6 | | √ | | | | |
| Rituximab 375 mg/m² | √ | | | | | |
| Cyclophosphamide 750 mg/m² | | √ | | | | |
| Anthracyclines*: for example, doxorubicin liposome 30-40 mg/m² | | √ | | | | |
| Vinca alkaloids*: for example, vincristine 1.4 mg/m² (maximum dose: 2 mg) | | √ | | | | |
| Prednisone 100 mg | | √ | √ | √ | √ | √ |

| | | | | | | |
|---|---|---|---|---|---|---|
| * The table lists anthracyclines and vinca alkaloids recommended in the R-CHOP regimen and their corresponding doses. If necessary, the investigator may substitute chemotherapeutic drugs of the same class with comparable efficacy, including epirubicin for doxorubicin liposome and vindesine for vincristine, in consultation with the sponsor and in accordance with institutional medical practices and guidelines. | | | | | | |

**Table 6. An administration schedule for combination therapy with the R-ICE/R-IC regimen in the Phase Ib dose escalation/expansion phase and Phase II cohort 2a/2b**

| | D1 | D2 | D3 | D4 |
|---|---|---|---|---|
| ADC-6 | | √ | | |
| Rituximab 375 mg/m² | √ | | | |
| Ifosfamide 5 g/m² | | | √ | |
| Carboplatin (calculated according to AUC = 5, single dose ≤ 800 mg) | | | √ | |
| Etoposide phosphate 100 mg/m² (except for use in combination with R-IC) | | √ | √ | √ |

### 4. Outcome evaluations

4.1. Effectiveness evaluation: Efficacy will be evaluated using the 2014 Lugano criteria for evaluating CT-based efficacy in lymphoma. Efficacy analysis involves: complete response rate (CRR), objective response rate (ORR), disease control rate (DCR), DoR, PFS, and OS.

4.2. Safety evaluation: Safety will be evaluated using the NCI-CTCAE 5.0 criteria.

### 5. Study results

As of November 19, 2024, a total of 37 subjects had been enrolled in the ADC-6 1.8 mg/kg plus R-GemOx cohort. The median number of lines of therapy was 1 (range: 1-3). Among these subjects, 70.3% had Lugano stage III-IV disease, 62.2% had disease refractory to the last line of therapy, and 56.8% had *de novo* refractory disease. Baseline characteristics are detailed in Table 1.

**Table 7. Baseline characteristics of patients treated with ADC-6 (1.8 mg/kg) plus R-GemOx**

| **Baseline characteristics** | **1.8 mg/kg (N=37)** |
|---|---|
| Sex | |
| Male | 20 (54.1) |
| Age (years), median (range) | 59 (22-76) |
| Number of prior lines of therapy | |
| 1 | 24 (64.9) |
| ≥2 | 13 (35.1) |
| DLBCL type | |
| DLBCL, GCB | 11 (29.7) |
| DLBCL, Non-GCB | 26 (70.3) |
| Lugano stage | |
| I-II | 11 (29.7) |
| III-IV | 26 (70.3) |
| IPI score | |
| Low risk (0-1) | 13 (35.1) |
| Moderate-to-low risk (2) | 9 (24.3) |
| Moderate-to-high risk (3) | 13 (35.1) |
| High risk (4-5) | 2 (5.4) |
| Refractory to last line* | 23 (62.2) |
| *de novo* refractory | 21 (56.8) |
| Baseline large lesion (any diameter ≥ 7.5 cm) | 4 (10.8) |

| | |
|---|---|
| * means that the best response to the last line of therapy was SD/PD, or PR/CR/NE with progression during treatment or within 6 months of completing treatment. | |

The treatment results show that among 37 DLBCL patients treated with ADC-6 (1.8 mg/kg) plus R-GemOx, 19 achieved a complete response (CR), and 8 achieved a partial response; the ORR was 73.0% (27/37), and the CRR was 51.4% (19/37). Among the 27 subjects who achieved a response, 23 (85.2%) achieved a response at the first efficacy assessment (end of C2, with every 3 weeks as one cycle), with the response continuing to deepen. CRs typically occurred at the second efficacy assessment (end of C4). All patients who achieved a response continued to have the response. The above effectiveness data are preliminary results, and the trial is still ongoing. Among the 37 subjects, no DLTs occurred. TEAEs occurring in ≥20% of the subjects were: decreased platelet count, anemia, elevated aspartate aminotransferase, decreased leukocyte count, decreased neutrophil count, elevated alanine aminotransferase, hypokalemia, nausea, hyperuricemia, vomiting, cough, elevated blood alkaline phosphatase, and decreased lymphocyte count. Grade ≥3 TEAEs occurring in ≥2 subjects included: decreased platelet count (29.7%), decreased neutrophil count (21.6%), decreased leukocyte count (24.3%), anemia (13.5%), decreased lymphocyte count (10.8%), and hypokalemia (5.4%).

From the above results, it can be seen that the ADC-6 + R-GemOx treatment regimen exhibited strong anti-tumor activity and rapid and lasting clinical responses. Moreover, no patient reported peripheral neurotoxicity during treatment, indicating a favorable safety profile and suggesting that this regimen can provide safe and effective clinical benefits to patients.

## Claims

1. Use of an anti-CD79b antibody-drug conjugate in combination with (1) an immunotherapeutic agent and/or (2) a chemotherapeutic agent in the manufacture of a medicament for treating a tumor,
wherein the anti-CD79b antibody-drug conjugate has a structure represented by the following formula: 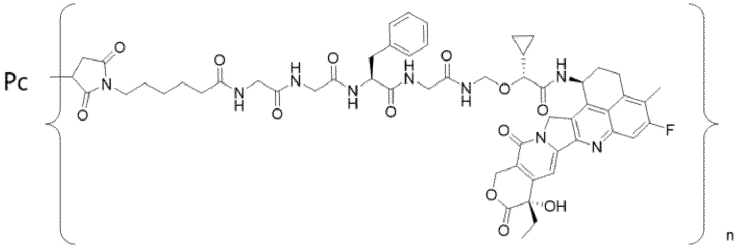
wherein Pc is an anti-CD79b antibody, and n is an integer or decimal from 1 to 10, preferably a decimal or integer from 1 to 6;
preferably, the tumor is preferably B-cell lymphoma.

2. The use according to claim 1, wherein a subject to which the anti-CD79b antibody-drug conjugate is administered experiences a reduced clinical adverse reaction, and the clinical adverse reaction is preferably peripheral neurotoxicity.

3. The use according to claim 1 or 2, wherein the anti-CD79b antibody comprises a heavy chain variable region (VH) and a light chain variable region (VL), wherein:
(1) the VH comprises a HCDR1, a HCDR2, and a HCDR3 from the sequence set forth in SEQ ID NO: 3, and the VL comprises a LCDR1, a LCDR2, and a LCDR3 from the sequence set forth in SEQ ID NO: 4;
(2) the VH comprises a HCDR1, a HCDR2, and a HCDR3 set forth in SEQ ID NO: 11, SEQ ID NO: 6, and SEQ ID NO: 7, respectively, and the VL comprises a LCDR1, a LCDR2, and a LCDR3 set forth in SEQ ID NO: 8, SEQ ID NO: 9, and SEQ ID NO: 10, respectively; or
(3) the VH comprises a HCDR1, a HCDR2, and a HCDR3 set forth in SEQ ID NO: 5, SEQ ID NO: 6, and SEQ ID NO: 7, respectively, and the VL comprises a LCDR1, a LCDR2, and a LCDR3 set forth in SEQ ID NO: 8, SEQ ID NO: 9, and SEQ ID NO: 10, respectively.

4. The use according to any one of claims 1-3, wherein the anti-CD79b antibody is a murine-derived antibody, a chimeric antibody, a humanized antibody, or a human antibody;
preferably, the anti-CD79b antibody comprises a VH comprising the sequence set forth in SEQ ID NO: 3 or a sequence having at least 80% or at least 90% homology therewith, and the anti-CD79b antibody comprises a VL comprising the sequence set forth in SEQ ID NO: 4 or a sequence having at least 80% or at least 90% homology therewith;
more preferably, the anti-CD79b antibody comprises a heavy chain comprising the sequence set forth in SEQ ID NO: 12 or a sequence having at least 80% or at least 90% homology therewith, and the anti-CD79b antibody comprises a light chain comprising the sequence set forth in SEQ ID NO: 13 or a sequence having at least 80% or at least 90% homology therewith.

5. The use according to any one of claims 1-4, wherein the anti-CD79b antibody-drug conjugate is administered at a dose of about 0.1 mg/kg to about 20 mg/kg, preferably about 1.0 mg/kg to about 10 mg/kg, preferably about 0.1 mg/kg, about 0.3 mg/kg, about 0.6 mg/kg, about 1.2 mg/kg, about 1.8 mg/kg, about 2.7 mg/kg, about 3.6 mg/kg, about 4.8 mg/kg, about 6.4 mg/kg, about 8.0 mg/kg, or about 10 mg/kg, and more preferably about 1.8 mg/kg, about 2.7 mg/kg, or about 3.6 mg/kg;
and/or
the anti-CD79b antibody-drug conjugate is administered at a frequency of once weekly, once every 2 weeks, once every 3 weeks, once every 4 weeks, once every 6 weeks, or once every 8 weeks.

6. The use according to any one of claims 1-5, wherein the immunotherapeutic agent is an anti-CD20 antibody, preferably rituximab;
the immunotherapeutic agent is preferably administered at a dose of about 1 mg/m² to about 1000 mg/m², more preferably about 375 mg/m², and the immunotherapeutic agent is preferably administered once weekly, once every 2 weeks, once every 3 weeks, once every 4 weeks, once every 6 weeks, or once every 8 weeks.

7. The use according to any one of claims 1-6, wherein the chemotherapeutic agent is selected from the group consisting of any one or any combination of the following:
gemcitabine, a platinum-based drug (e.g., oxaliplatin or carboplatin), cyclophosphamide, an anthracycline (e.g., doxorubicin), a vinca alkaloid (e.g., vincristine), prednisone, ifosfamide, and etoposide phosphate;
preferably, the chemotherapeutic agent comprises any one of the following combinations:
(a) gemcitabine and oxaliplatin;
(b) cyclophosphamide, an anthracycline (e.g., doxorubicin), a vinca alkaloid (e.g., vincristine), and prednisone;
(c) ifosfamide, carboplatin, and etoposide phosphate; and
(d) ifosfamide and carboplatin;
preferably, gemcitabine is administered at a dose of about 5 mg/m² to about 2000 mg/m², more preferably about 1000 mg/m²;
oxaliplatin is administered at a dose of about 1 mg/m² to about 500 mg/m², more preferably about 100 mg/m²;
cyclophosphamide is administered at a dose of about 1 mg/m² to about 1000 mg/m², more preferably about 750 mg/m²;
the anthracycline is at about 1 mg/m² to about 500 mg/m², more preferably 30-40 mg/m²; the vinca alkaloid is administered at a dose of about 0.1 mg/m² to about 100 mg/m², more preferably about 1.4 mg/m², and more preferably no more than 2 mg;
prednisone is administered at a dose of about 1 mg/time to about 1000 mg/time, more preferably about 100 mg/time;
ifosfamide is administered at a dose of about 0.1 mg/m² to about 100 mg/m², more preferably about 5 mg/m²;
carboplatin is administered at a dose of about 1 mg to about 2000 mg, more preferably ≤800 mg;
etoposide phosphate is administered at a dose of about 1 mg/m² to about 500 mg/m²/time, more preferably about 100 mg/m²/time;
an administration cycle of the chemotherapeutic agent is preferably every 1 week, every 2 weeks, every 3 weeks, every 4 weeks, every 6 weeks, or every 8 weeks;
more preferably, gemcitabine, oxaliplatin, cyclophosphamide, the anthracycline, the vinca alkaloid, ifosfamide, and carboplatin are administered at a frequency of once every 2 weeks, once every 3 weeks, or once every 4 weeks, prednisone is administered at a frequency of 3, 4, or 5 times every 3 weeks, and etoposide phosphate is administered at a frequency of 3 or 4 times every 3 weeks.

8. The use according to any one of claims 1-7, wherein the (1) immunotherapeutic agent and/or (2) chemotherapeutic agent is selected from the group consisting of any one of the following combinations:
(a) rituximab, gemcitabine, and oxaliplatin;
(b) rituximab, cyclophosphamide, an anthracycline (e.g., doxorubicin), a vinca alkaloid (e.g., vincristine), and prednisone;
(c) rituximab, ifosfamide, carboplatin, and etoposide phosphate; and
(d) rituximab, ifosfamide, and carboplatin.

9. The use according to any one of claims 1-8, wherein any one of the following pharmaceutical combinations is administered every 2 weeks, every 3 weeks, or every 4 weeks:
(1) about 1.0 mg/kg to about 10 mg/kg of the anti-CD79b antibody-drug conjugate, about 1 mg/m² to about 1000 mg/m² of rituximab, about 5 mg/m² to about 2000 mg/m² of gemcitabine, and about 1-500 mg/m² of oxaliplatin;
(2) about 1.0 mg/kg to about 10 mg/kg of the anti-CD79b antibody-drug conjugate, about 1 mg/m² to about 1000 mg/m² of rituximab, about 1 mg/m² to about 1000 mg/m² of cyclophosphamide, about 1 mg/m² to about 500 mg/m² of an anthracycline, about 0.1 mg/m² to about 100 mg/m² of a vinca alkaloid, and about 1 mg/time to about 1000 mg/time of prednisone;
(3) about 1.0 mg/kg to about 10 mg/kg of the anti-CD79b antibody-drug conjugate, about 1 mg/m² to about 1000 mg/m² of rituximab, about 0.1 mg/m² to about 100 mg/m² of ifosfamide, about 1 mg to about 2000 mg of carboplatin, and about 1 mg/m²/time to about 500 mg/m²/time of etoposide phosphate;
(4) about 1.0 mg/kg to about 10 mg/kg of the anti-CD79b antibody-drug conjugate, about 1 mg/m² to about 1000 mg/m² of rituximab, about 0.1 mg/m² to about 100 mg/m² of ifosfamide, and about 1 mg to about 2000 mg of carboplatin;
(5) about 0.1 mg/kg, about 0.3 mg/kg, about 0.6 mg/kg, about 1.2 mg/kg, about 1.8 mg/kg, about 2.7 mg/kg, about 3.6 mg/kg, about 4.8 mg/kg, about 6.4 mg/kg, about 8.0 mg/kg, or about 10 mg/kg of the anti-CD79b antibody-drug conjugate, about 375 mg/m² of rituximab, about 1000 mg/m² of gemcitabine, and about 100 mg/m² of oxaliplatin;
(6) about 0.1 mg/kg, about 0.3 mg/kg, about 0.6 mg/kg, about 1.2 mg/kg, about 1.8 mg/kg, about 2.7 mg/kg, about 3.6 mg/kg, about 4.8 mg/kg, about 6.4 mg/kg, about 8.0 mg/kg, or about 10 mg/kg of the anti-CD79b antibody-drug conjugate, about 375 mg/m² of rituximab, about 750 mg/m² of cyclophosphamide, about 30-40 mg/m² of an anthracycline (e.g., doxorubicin), about 1.4 mg/m² of a vinca alkaloid (e.g., vincristine), and about 100 mg of prednisone;
(7) about 0.1 mg/kg, about 0.3 mg/kg, about 0.6 mg/kg, about 1.2 mg/kg, about 1.8 mg/kg, about 2.7 mg/kg, about 3.6 mg/kg, about 4.8 mg/kg, about 6.4 mg/kg, about 8.0 mg/kg, or about 10 mg/kg of the anti-CD79b antibody-drug conjugate, about 375 mg/m² of rituximab, about 5 mg/m² of ifosfamide, ≤800 mg of carboplatin, and about 100 mg/m² of etoposide phosphate; and
(8) about 0.1 mg/kg, about 0.3 mg/kg, about 0.6 mg/kg, about 1.2 mg/kg, about 1.8 mg/kg, about 2.7 mg/kg, about 3.6 mg/kg, about 4.8 mg/kg, about 6.4 mg/kg, about 8.0 mg/kg, or about 10 mg/kg of the anti-CD79b antibody-drug conjugate, about 375 mg/m² of rituximab, about 5 mg/m² of ifosfamide, and ≤800 mg of carboplatin.

10. The use according to claim 9, wherein rituximab is administered before the anti-CD79b antibody-drug conjugate; preferably:
rituximab is administered on day 1 of each administration cycle, and the anti-CD79b antibody-drug conjugate, gemcitabine, and oxaliplatin are administered on day 2;
rituximab is administered on day 1 of each administration cycle, the anti-CD79b antibody-drug conjugate, cyclophosphamide, the anthracycline, and the vinca alkaloid are administered on day 2, and prednisone is administered on days 2, 3, 4, 5, and 6;
rituximab is administered on day 1 of each administration cycle, the anti-CD79b antibody-drug conjugate is administered on day 2, ifosfamide and carboplatin are administered on day 3, and etoposide phosphate is administered on days 2, 3, and 4; or
rituximab is administered on day 1 of each administration cycle, the anti-CD79b antibody-drug conjugate is administered on day 2, and ifosfamide and carboplatin are administered on day 3.

11. The use according to any one of claims 1-10, wherein the B-cell lymphoma is diffuse large B-cell lymphoma, follicular lymphoma, and marginal zone lymphoma; preferably, the diffuse large B-cell lymphoma is selected from the group consisting of diffuse large B-cell lymphoma that is relapsed or refractory or has not been treated with prior lymphoma-related systemic anti-tumor therapy.

12. The use according to any one of claims 1-11, wherein a subject having the tumor has received at least one prior line of anti-tumor therapy; preferably, the subject has received at least one prior line of anti-tumor therapy and did not achieve a response after the last line of therapy or experienced disease progression and did not achieve a response after treatment or experienced disease progression after treatment;
preferably, the subject is given the anti-CD79b antibody-drug conjugate, rituximab, gemcitabine, and oxaliplatin, and has not received prior treatment with gemcitabine; or the subject is given the anti-CD79b antibody-drug conjugate, ifosfamide, and carboplatin, and has not received prior treatment with ifosfamide; or
the subject is given the anti-CD79b antibody-drug conjugate, etoposide phosphate, ifosfamide, and carboplatin, and has not received prior treatment with ifosfamide.

13. The use according to any one of claims 1-12, wherein a subject having the tumor has Lugano stage I-II or III-IV disease, and/or has an IPI score of 0-1, 2, 3, or 4-5.

14. A pharmaceutical composition, comprising a) an anti-CD79b antibody-drug conjugate and b) an immunotherapeutic agent and/or a chemotherapeutic agent, wherein the anti-CD79b antibody-drug conjugate has a structure represented by the following formula: 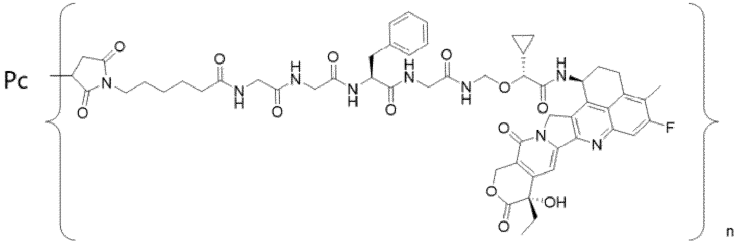
wherein Pc is an anti-CD79b antibody, and n is an integer or decimal from 1 to 10, preferably a decimal or integer from 1 to 6;
preferably, the anti-CD79b antibody-drug conjugate, the immunotherapeutic agent, and the chemotherapeutic agent are as defined in any one of claims 1-10;
preferably, the pharmaceutical composition is used for treating a subject having a tumor, wherein the tumor is preferably B-cell lymphoma, and is more preferably as defined in any one of claims 11-13.

15. A method for treating a tumor, comprising administering to a subject in need thereof a therapeutically effective amount of an anti-CD79b antibody-drug conjugate, wherein:
the anti-CD79b antibody-drug conjugate is administered at a dose of about 0.1 mg/kg to about 20 mg/kg, preferably about 1.0 mg/kg to about 10 mg/kg;
the anti-CD79b antibody-drug conjugate has a structure represented by the following formula: 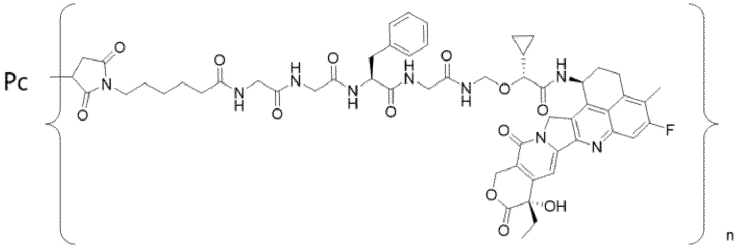
wherein Pc is an anti-CD79b antibody, and n is an integer or decimal from 1 to 10, preferably a decimal or integer from 1 to 6; the tumor is preferably B-cell lymphoma, and is more preferably as defined in any one of claims 11-13;
preferably, the method further comprises administering an immunotherapeutic agent and/or a chemotherapeutic agent;
preferably, the anti-CD79b antibody-drug conjugate, the immunotherapeutic agent, and the chemotherapeutic agent are as defined in any one of claims 1-10.

16. A method for palliating an adverse reaction caused by an anti-CD79b antibody-drug conjugate in a subject, comprising administering to a subject in need thereof an antibody-drug conjugate having the following structure: 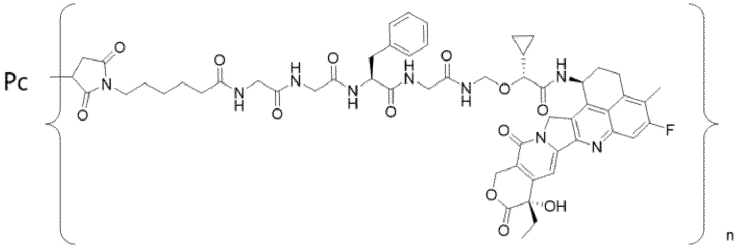
wherein Pc is an anti-CD79b antibody, and n is an integer or decimal from 1 to 10, preferably a decimal or integer from 1 to 6;
preferably, the method further comprises administering an immunotherapeutic agent and/or a chemotherapeutic agent;
preferably, the adverse reaction is neurotoxicity, more preferably peripheral neurotoxicity (PN);
preferably, the subject is a patient with a tumor, wherein the tumor is preferably B-cell lymphoma, and is more preferably as defined in any one of claims 11-13;
preferably, the anti-CD79b antibody-drug conjugate, the immunotherapeutic agent, and the chemotherapeutic agent are as defined in any one of claims 1-10.
